# EUROPEAN PATENT APPLICATION

(11) **EP 4 516 314 A1**
(43) Date of publication of application: **05.03.2025**
(21) Application number: 23795433.4
(22) Date of filing: 25.04.2023
(51) Int. Cl.: A61K 39/395, C07K 16/28, A61P 35/00, A61P 35/04

(54) **COMBINATION OF ANTI-PD-1 ANTIBODY AND ANTI-EGFR ANTIBODY, AND USE THEREOF IN TREATMENT OF HEAD AND NECK SQUAMOUS CELL CARCINOMA**

(30) Priority: 26.04.2022 CN 202210493473
(71) Applicant: Topalliance Biosciences Inc., 20850 Rockville, MD (US)
(72) Inventor: YAO, Sheng, Shanghai 201210 (CN); FENG, Hui, Shanghai 201210 (CN)
(74) Representative: V.O.
(86) International application number: PCT/CN2023/090687
(87) International publication number: WO 2023/208001

(57) **Abstract**

Provided are a combination of an anti-PD-1 antibody and an anti-EGFR antibody and the use thereof in the treatment of head and neck squamous cell carcinoma. Specifically, provided is a drug combination comprising an anti-PD-1 antibody or an antigen-binding fragment thereof and an anti-EGFR antibody or an antigen-binding fragment thereof. The drug combination exhibits good curative effect in the treatment of head and neck squamous cell carcinoma.

## Description

### TECHNICAL FIELD

The present disclosure relates to the technical field of pharmaceutical applications, and particularly relates to a combination of an anti-PD-1 antibody or an antigen-binding fragment thereof and an anti-EGFR antibody or an antigen-binding fragment thereof, and use thereof in the preparation of a medicament for treating head and neck squamous cell carcinoma.

### BACKGROUND

Head and neck tumors refer to tumors occurring in the mouth, nose, pharynx, larynx, and other areas. More than 90% of these tumors are squamous cell carcinoma and its variants, collectively known as head and neck squamous cell carcinoma (HNSCC).

HNSCC is the sixth most common cancer worldwide, with 890,000 new cases diagnosed and 450,000 deaths in 2018. The incidence of HNSCC continues to rise and is expected to increase by 30% by 2030. The high prevalence of HNSCC in countries and regions such as Southeast Asia and Australia is associated with the consumption of specific carcinogen-containing products, while the increase in oropharyngeal HPV infection rate results in a high prevalence of HNSCC in the United States and Western Europe. Generally, the risk of developing HNSCC in men is 2-4 times higher than in women. The median age at diagnosis for non-virus-related HNSCC is 66 years, while the median ages at diagnosis for HPV-related oropharyngeal carcinoma and EBV-related nasopharyngeal carcinoma are 53 and 50 years, respectively.

More than 65% of head and neck squamous cell carcinomas are already recurrent or metastatic at the time of diagnosis. Among patients with locally advanced HNSCC, 15-40% will still experience recurrence or metastasis, with a 5-year survival rate of less than 50%. Recurrent or metastatic head and neck squamous cell carcinoma has a poor prognosis and a short survival time. Since its anatomical locations are mainly located in vital organs, it can also damage the appearance, basic physiological functions, sensory functions, language function and the like of a patient, thus affecting the patient's quality of life.

For the first-line treatment of head and neck squamous cell carcinomas (excluding nasopharyngeal carcinoma), platinum-based regimens (platinum in combination with fluorouracil or taxane ± cetuximab) are the standard therapies recommended by current domestic guidelines. There is currently no standard therapy for recurrent or metastatic head and neck squamous cell carcinoma that has failed the first-line platinum-based treatment regimen. For example, for patients with PD-L1 ≥ 1%, nivolumab can be used for treatment. However, for people who have not been screened for biomarkers, current treatment is still mainly based on single-agent chemotherapy such as methotrexate, docetaxel or the like. The response rate is lower than 10%, and the overall survival is 6-7 months, indicating very limited efficacy.

Currently, immunotherapy for recurrent or metastatic head and neck squamous cell carcinoma that has failed the first-line platinum-based treatment regimen mainly includes immunoregulators, immune checkpoint blockers (PD-1/PD-L1 and CTLA-4 blockers, etc.), tumor vaccines, cellular immunotherapy (cytokine-induced killer cells (CIK)), and the like. Among them, programmed death receptor-1 (PD-1) is an inhibitory receptor of the immunoglobulin family on the surface of activated T lymphocytes, and its ligands are the B7 homologous proteins programmed death ligand-1 (PD-L1) (also known as B7-H1) and programmed death ligand-2 (PD-L2) (also known as B7-DC). The binding of PD-1/PD-L1 plays an important role in down-regulating T cell activation and maintaining peripheral immune tolerance. Therefore, tumor cells express PD-L1, which further interacts with PD-1 to inhibit T cell activation, allowing the tumor cells to escape from killing by immune cells. By blocking such immune checkpoints, the proliferation, survival, and killing activity of T cells can be enhanced, achieving tumor immunotherapy effects.

Epidermal growth factor receptor (EGFR) belongs to the tyrosine kinase receptor family. Abnormal EGFR activation in cancer cells can be induced by various mechanisms, including gene amplification, point mutations, deletions, autocrine ligand-receptor stimulation, and the like. By establishing a non-inflammatory tumor microenvironment, the activity of CD8+ T cells can be reduced and the immunosuppressive function of Treg cells can be enhanced, thereby promoting tumor development and progression. Monoclonal antibodies targeting EGFR can block the binding of EGF and other ligands to EGFR, preventing dimerization of EGFR and thus inhibiting ligand-induced activation of this receptor tyrosine kinase. This leads to the inhibition of cell growth, the induction of cell apoptosis, and the reduction in the synthesis of matrix metalloproteinases and vascular endothelial growth factors. Monoclonal antibodies targeting EGFR also prevent interaction with its ligands by eliminating receptors from the cell surface, stimulating internalization and eventual degradation of EGFR. In addition, monoclonal antibodies targeting EGFR can mediate antibody-dependent, cell-mediated cytotoxicity; this process depends on both the affinity of the monoclonal antibodies for the extracellular domain of EGFR and the expression level of cellular EGFR.

In the process of HNSCC treatment, the therapeutic effect of monotherapy has certain limitations. Therefore, exploring more effective treatment means to further extend the survival of patients with recurrent or metastatic head and neck squamous cell carcinoma and improve their quality of life has become one of the clinical needs to be addressed urgently.

### SUMMARY

In a first aspect, the present disclosure provides a pharmaceutical combination, comprising an anti-PD-1 antibody or an antigen-binding fragment thereof and an anti-EGFR antibody or an antigen-binding fragment thereof.

In one or more embodiments, the anti-PD-1 antibody or the antigen-binding fragment thereof described herein comprises LCDR1 with the amino acid sequence set forth in SEQ ID NO: 1 or an amino acid sequence having 1, 2, or 3 amino acid differences compared to SEQ ID NO: 1, LCDR2 with the amino acid sequence set forth in SEQ ID NO: 2 or an amino acid sequence having 1, 2, or 3 amino acid differences compared to SEQ ID NO: 2, LCDR3 with the amino acid sequence set forth in SEQ ID NO: 3 or an amino acid sequence having 1, 2, or 3 amino acid differences compared to SEQ ID NO: 3, HCDR1 with the amino acid sequence set forth in SEQ ID NO: 4 or an amino acid sequence having 1, 2, or 3 amino acid differences compared to SEQ ID NO: 4, HCDR2 with the amino acid sequence set forth in SEQ ID NO: 5 or an amino acid sequence having 1, 2, or 3 amino acid differences compared to SEQ ID NO: 5, and HCDR3 with the amino acid sequence set forth in SEQ ID NO: 6 or an amino acid sequence having 1, 2, or 3 amino acid differences compared to SEQ ID NO: 6.

In one or more embodiments, the anti-PD-1 antibody or the antigen-binding fragment thereof described herein comprises a light chain variable region having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 7, and a heavy chain variable region having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 8.

In one or more embodiments, the anti-PD-1 antibody or the antigen-binding fragment thereof described herein comprises a light chain variable region with the amino acid sequence set forth in SEQ ID NO: 7 and a heavy chain variable region with the amino acid sequence set forth in SEQ ID NO: 8.

In one or more embodiments, the anti-PD-1 antibody or the antigen-binding fragment thereof described herein comprises a light chain having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 9, and a heavy chain having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 10.

In one or more embodiments, the anti-PD-1 antibody described herein comprises a light chain with the amino acid sequence set forth in SEQ ID NO: 9 and a heavy chain with the amino acid sequence set forth in SEQ ID NO: 10.

The anti-PD-1 antibody in the pharmaceutical combination of the present disclosure may be any antibody that is capable of specifically binding to PD-1 and blocking or inhibiting the binding of PD-1 to its receptor PD-L1.

In one or more embodiments, the anti-PD-1 antibody described herein is selected from one or more of nivolumab or a biosimilar thereof, pembrolizumab or a biosimilar thereof, toripalimab or a biosimilar thereof, sintilimab or a biosimilar thereof, camrelizumab or a biosimilar thereof, tislelizumab or a biosimilar thereof, cemiplimab or a biosimilar thereof, zimberelimab or a biosimilar thereof, penpulimab or a biosimilar thereof, and serplulimab or a biosimilar thereof, preferably toripalimab or a biosimilar thereof.

In one or more embodiments, the anti-EGFR antibody or the antigen-binding fragment thereof described herein comprises LCDR1 with the amino acid sequence set forth in SEQ ID NO: 11 or an amino acid sequence having 1, 2, or 3 amino acid differences compared to SEQ ID NO: 11, LCDR2 with the amino acid sequence set forth in SEQ ID NO: 12 or an amino acid sequence having 1, 2, or 3 amino acid differences compared to SEQ ID NO: 12, LCDR3 with the amino acid sequence set forth in SEQ ID NO: 13 or an amino acid sequence having 1, 2, or 3 amino acid differences compared to SEQ ID NO: 13, HCDR1 with the amino acid sequence set forth in SEQ ID NO: 14 or an amino acid sequence having 1, 2, or 3 amino acid differences compared to SEQ ID NO: 14, HCDR2 with the amino acid sequence set forth in SEQ ID NO: 15 or an amino acid sequence having 1, 2, or 3 amino acid differences compared to SEQ ID NO: 15, and HCDR3 with the amino acid sequence set forth in SEQ ID NO: 16 or an amino acid sequence having 1, 2, or 3 amino acid differences compared to SEQ ID NO: 16.

In one or more embodiments, the anti-EGFR antibody or the antigen-binding fragment thereof described herein comprises a light chain variable region having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 17, and a heavy chain variable region having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 18.

In one or more embodiments, the anti-EGFR antibody or the antigen-binding fragment thereof described herein comprises a light chain variable region with the amino acid sequence set forth in SEQ ID NO: 17 and a heavy chain variable region with the amino acid sequence set forth in SEQ ID NO: 18.

In one or more embodiments, the anti-EGFR antibody or the antigen-binding fragment thereof described herein comprises a light chain having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 19, and a heavy chain having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 20.

In one or more embodiments, the anti-EGFR antibody described herein comprises a light chain with the amino acid sequence set forth in SEQ ID NO: 19 and a heavy chain with the amino acid sequence set forth in SEQ ID NO: 20.

The anti-EGFR antibody in the pharmaceutical combination of the present disclosure may be any antibody that is capable of specifically binding to EGFR and blocking or inhibiting the binding of EGF to its receptor EGFR.

In one or more embodiments, the anti-EGFR antibody described herein is selected from one or more of necitumumab or a biosimilar thereof, nimotuzumab or a biosimilar thereof, panitumumab or a biosimilar thereof, and cetuximab or a biosimilar thereof, preferably cetuximab or a biosimilar thereof.

In one or more embodiments, the pharmaceutical combination described herein comprises toripalimab or a biosimilar thereof and cetuximab or a biosimilar thereof.

In one or more embodiments, the pharmaceutical combination described herein comprises toripalimab and cetuximab.

In one or more embodiments, the pharmaceutical combination comprises 1 dose of the anti-PD-1 antibody or the antigen-binding fragment thereof (preferably toripalimab) and 3 doses of the anti-EGFR antibody or the antigen-binding fragment thereof (preferably cetuximab), wherein the anti-PD-1 antibody or the antigen-binding fragment thereof and the anti-EGFR antibody or the antigen-binding fragment thereof are provided in separate formulations or in an integral package. Preferably, the 1 dose of the formulation of the anti-PD-1 antibody or the antigen-binding fragment thereof comprises 240 mg of the anti-PD-1 antibody or the antigen-binding fragment thereof; among the 3 doses of the formulation of the anti-EGFR antibody or the antigen-binding fragment thereof, 1 dose of the formulation comprises the anti-EGFR antibody or the antigen-binding fragment thereof sufficient for administration at a daily dose of 400 mg/m², while the other 2 doses are sufficient for administration at a daily dose of 250 mg/m², or each of the 3 doses of the formulation of the anti-EGFR antibody or the antigen-binding fragment thereof comprises the anti-EGFR antibody or the antigen-binding fragment thereof sufficient for administration at a daily dose of 250 mg/m². Preferably, the anti-PD-1 antibody is toripalimab, and the anti-EGFR antibody is cetuximab.

In a second aspect, the present disclosure provides use of the pharmaceutical combination described above in the preparation of a medicament for preventing or treating head and neck squamous cell carcinoma (HNSCC) in a patient, comprising administering to a subject a therapeutically effective amount of the pharmaceutical combination as described in any one of the above embodiments. In one or more embodiments, the head and neck squamous cell carcinoma in the use is recurrent or metastatic head and neck squamous cell carcinoma, preferably recurrent or metastatic head and neck squamous cell carcinoma that has failed a previous first-line platinum-based chemotherapy regimen. In one or more embodiments, the patient has a PD-L1 expression of ≥ 1 (i.e., PD-L1 CPS ≥ 1) as detected by immunohistochemistry. In one or more embodiments, the head and neck squamous cell carcinoma in the use has ≥ 1 measurable lesion according to RECIST v1.1 criteria.

In a third aspect, the present disclosure provides use of an anti-PD-1 antibody or an antigen-binding fragment thereof in the preparation of a medicament or kit for preventing or treating head and neck squamous cell carcinoma (HNSCC) in a patient, or use of a combination of an anti-PD-1 antibody or an antigen-binding fragment thereof and an anti-EGFR antibody or an antigen-binding fragment thereof in the preparation of a medicament or kit for preventing or treating head and neck squamous cell carcinoma in a patient.

In a fourth aspect, the present disclosure provides a method for preventing or treating head and neck squamous cell carcinoma in a patient, comprising administering to an individual in need a therapeutically effective amount of an anti-PD-1 antibody or an antigen-binding fragment thereof, or a combination of an anti-PD-1 antibody or an antigen-binding fragment thereof and an anti-EGFR antibody or an antigen-binding fragment thereof.

In a fifth aspect, the present disclosure provides an anti-PD-1 antibody or an antigen-binding fragment thereof, or a combination of an anti-PD-1 antibody or an antigen-binding fragment thereof and an anti-EGFR antibody or an antigen-binding fragment thereof, for use in the prevention or treatment of head and neck squamous cell carcinoma in a patient.

In one or more embodiments, the head and neck squamous cell carcinoma described herein is recurrent or metastatic head and neck squamous cell carcinoma, preferably recurrent or metastatic head and neck squamous cell carcinoma that has failed a previous first-line platinum-based chemotherapy regimen.

In one or more embodiments, the patient has a PD-L1 CPS of ≥ 1.

In one or more embodiments, the head and neck squamous cell carcinoma described herein has ≥ 1 measurable lesion according to RECIST v1.1 criteria.

In one or more embodiments, in the second aspect, the third aspect, the fourth aspect and the fifth aspect of the present disclosure, the anti-PD-1 antibody or the antigen-binding fragment thereof is the anti-PD-1 antibody or the antigen-binding fragment thereof as described in any one of the embodiments herein.

In one or more embodiments, in the second aspect, the third aspect, the fourth aspect and the fifth aspect of the present disclosure, the anti-EGFR antibody or the antigen-binding fragment thereof is the anti-EGFR antibody or the antigen-binding fragment thereof as described in any one of the embodiments herein.

In one or more embodiments, the anti-PD-1 antibody or the antigen-binding fragment thereof described herein is administered alone.

In one or more embodiments, the anti-PD-1 antibody or the antigen-binding fragment thereof described herein is administered at a single dose of about 0.1 mg/kg individual body weight to about 10.0 mg/kg individual body weight, preferably about 1.0 mg/kg individual body weight to about 10.0 mg/kg individual body weight, such as about 0.1 mg/kg individual body weight, about 0.3 mg/kg individual body weight, about 1 mg/kg individual body weight, about 2 mg/kg individual body weight, about 3 mg/kg individual body weight, about 5 mg/kg individual body weight, or about 10 mg/kg individual body weight, or of a fixed dose of about 120 mg to about 480 mg, preferably a fixed dose of about 120 mg to about 360 mg, such as a fixed dose of about 120 mg, about 240 mg, about 360 mg, or about 480 mg.

In one or more embodiments, the anti-PD-1 antibody or the antigen-binding fragment thereof described herein is administered at a frequency of about once every week, once every two weeks, once every three weeks, once every four weeks, or once a month, preferably once every two weeks or once every three weeks.

In one or more embodiments, the anti-PD-1 antibody or the antigen-binding fragment thereof described herein is administered once every two or three weeks, at a dose of about 1 mg/kg individual body weight, about 3 mg/kg individual body weight, about 5 mg/kg individual body weight, or about 10 mg/kg individual body weight, or of a fixed dose of about 240 mg, a fixed dose of about 360 mg, or a fixed dose of about 480 mg.

In one or more embodiments, the anti-PD-1 antibody or the antigen-binding fragment thereof described herein is administered in a liquid dosage form such as an injection, via a parenteral route, for example, by intravenous infusion.

In one or more embodiments, the anti-PD-1 antibody or the antigen-binding fragment thereof described herein is administered in cycles of one week, two weeks, three weeks, one month, two months, three months, four months, five months, half a year, one year, two years, or longer; optionally, the administration cycles have identical or different durations, and are at identical or different intervals.

In one or more embodiments, the anti-PD-1 antibody or the antigen-binding fragment thereof described herein is administered in combination with the anti-EGFR antibody or the antigen-binding fragment thereof.

In one or more embodiments, the anti-PD-1 antibody or the antigen-binding fragment thereof described herein is administered at a single dose of about 0.1 mg/kg individual body weight to about 10.0 mg/kg individual body weight, preferably about 1.0 mg/kg individual body weight to about 10.0 mg/kg individual body weight, such as about 0.1 mg/kg individual body weight, about 0.3 mg/kg individual body weight, about 1 mg/kg individual body weight, about 2 mg/kg individual body weight, about 3 mg/kg individual body weight, about 5 mg/kg individual body weight, or about 10 mg/kg individual body weight, or of a fixed dose of about 120 mg to about 480 mg, preferably a fixed dose of about 120 mg to about 360 mg, such as a fixed dose of about 120 mg, about 240 mg, about 360 mg, or about 480 mg, preferably a fixed dose of about 240 mg.

In one or more embodiments, the anti-EGFR antibody or the antigen-binding fragment thereof described herein is administered at a single dose of about 100 mg/m² individual body surface area to about 500 mg/m² individual body surface area, preferably 200 mg/m² individual body surface area to about 500 mg/m² individual body surface area, such as about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area, preferably about 200 mg/m² individual body surface area, about 250 mg/m² individual body surface area, about 300 mg/m² individual body surface area, about 350 mg/m² individual body surface area, or about 400 mg/m² individual body surface area.

In one or more embodiments, the anti-PD-1 antibody or the antigen-binding fragment thereof described herein is administered at a frequency of about once every week, once every two weeks, once every three weeks, once every four weeks, or once a month, preferably once every three weeks.

In one or more embodiments, the anti-EGFR antibody or the antigen-binding fragment thereof described herein is administered at a frequency of about once every week, once every two weeks, once every three weeks, once every four weeks, or once a month, preferably once every week.

In one or more embodiments, the anti-PD-1 antibody or the antigen-binding fragment thereof is administered once every three weeks at a fixed dose of about 240 mg; and the anti-EGFR antibody or the antigen-binding fragment thereof is administered once every week, at a dose of about 400 mg/m² individual body surface area in the first cycle, and about 200 mg/m² individual body surface area or 250 mg/m² individual body surface area in each subsequent administration cycle.

In one or more embodiments, the anti-PD-1 antibody or the antigen-binding fragment thereof or the anti-EGFR antibody or the antigen-binding fragment thereof may be administered in cycles of one week, two weeks, three weeks, one month, two months, three months, four months, five months, half a year, one year, two years, or longer; optionally, the administration cycles may have identical or different durations, and may be at identical or different intervals.

In one or more embodiments, in the second aspect, the third aspect, the fourth aspect and the fifth aspect of the present disclosure, the anti-PD-1 antibody or the antigen-binding fragment thereof and the anti-EGFR antibody or the antigen-binding fragment thereof are administered in a liquid dosage form such as an injection, via a parenteral route, for example, by intravenous infusion.

In a sixth aspect, the present disclosure provides a kit, comprising:
one or more single dosage units of an anti-PD-1 antibody or an antigen-binding fragment thereof, wherein preferably, the anti-PD-1 antibody or the antigen-binding fragment thereof is as described in any one of the embodiments herein, and more preferably, the anti-PD-1 antibody is toripalimab or a biosimilar thereof; or
one or more single dosage units of an anti-PD-1 antibody or an antigen-binding fragment thereof and one or more single dosage units of an anti-EGFR antibody or an antigen-binding fragment thereof, wherein preferably, the anti-PD-1 antibody or the antigen-binding fragment thereof is as described in any one of the embodiments herein, and preferably, the anti-EGFR antibody or the antigen-binding fragment thereof is as described in any one of the embodiments herein, and more preferably, the anti-PD-1 antibody is toripalimab or a biosimilar thereof, and the anti-EGFR antibody is cetuximab or a biosimilar thereof.

In one or more embodiments, the kit comprises:
(I) one or more single dosage units of the anti-PD-1 antibody or the antigen-binding fragment thereof, wherein the single dosage unit comprises the anti-PD-1 antibody or the antigen-binding fragment thereof in an amount of a fixed dose of about 120 mg to about 480 mg, such as a fixed dose of about 120 mg, a fixed dose of about 240 mg, a fixed dose of about 360 mg, or a fixed dose of about 480 mg, or comprises the anti-PD-1 antibody or the antigen-binding fragment thereof in an amount sufficient for administration to the patient at a single dose of about 1.0 mg/kg individual body weight to about 10.0 mg/kg individual body weight, about 1 mg/kg individual body weight, about 3 mg/kg individual body weight, about 5 mg/kg individual body weight, or about 10 mg/kg individual body weight; or
(II) one or more single dosage units of the anti-PD-1 antibody or the antigen-binding fragment thereof, wherein the single dosage unit comprises the anti-PD-1 antibody or the antigen-binding fragment thereof in an amount of a fixed dose of about 120 mg to about 480 mg, preferably a fixed dose of about 120 mg to about 360 mg, such as about 120 mg, about 240 mg, about 360 mg, or about 480 mg, preferably a fixed dose of about 240 mg; and one or more single dosage units of the anti-EGFR antibody or the antigen-binding fragment thereof, wherein the single dosage unit comprises the anti-EGFR antibody or the antigen-binding fragment thereof in an amount sufficient for administration to the patient at a single dose of about 100 mg/m² individual body surface area to about 500 mg/m² individual body surface area, preferably 200 mg/m² individual body surface area to about 500 mg/m² individual body surface area, such as about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area, preferably about 200 mg/m² individual body surface area, about 250 mg/m² individual body surface area, about 300 mg/m² individual body surface area, about 350 mg/m² individual body surface area, or about 400 mg/m² individual body surface area.

In one or more embodiments, the kit described herein further comprises instructions for medication use.

In one or more embodiments, the kit comprises the pharmaceutical combination as described in any one of the embodiments herein.

### BRIEF DESCRIPTION OF THE DRAWINGS

FIG. 1: changes in patient tumor burden from baseline evaluated according to RECIST v1.1 and efficacy evaluation results (n = 45).
FIG. 2: changes in patient tumor burden from baseline evaluated according to RECIST v1.1 (n = 45).

### DETAILED DESCRIPTION

The present disclosure relates to a method for treating a malignant tumor. The method of the present disclosure comprises administering to a patient in need an anti-PD-1 antibody or an antigen-binding fragment thereof or a combination thereof, and an anti-EGFR antibody or an antigen-binding fragment thereof or a combination thereof. The malignant tumor described herein is head and neck squamous cell carcinoma.

### Terminology

In order to facilitate the understanding of the present disclosure, some technical and scientific terms are specifically defined below. Unless otherwise specifically stated herein, all technical and scientific terms used herein have the meaning as commonly understood by those of ordinary skill in the art to which the present disclosure belongs.

As used in this specification and the appended claims, the singular forms "a", "an" and "the" include plural referents, unless otherwise specifically stated. Thus, for example, reference to "a polypeptide" includes a combination of two or more polypeptides and the like.

"Administer", "give", and "treat" refers to introducing a composition comprising a therapeutic agent into a subject using any one of a variety of methods or delivery systems known to those skilled in the art. Routes of administration of the anti-PD-1 antibody include intravenous, intramuscular, subcutaneous, intraperitoneal, spinal or other parenteral routes of administration, such as injection or infusion. "Parenteral administration" refers to modes of administration apart from enteral or topical administration, typically by injection, including, but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion, and *in vivo* electroporation.

The "adverse event" (AE) described herein is any adverse and often unintended or undesirable sign, symptom, or disease associated with the use of medical treatment. For example, an adverse event may be associated with the activation of the immune system or the amplification of immune system cells in response to treatment. The medical treatment may have one or more related AEs, and the AEs may have identical or different severity levels.

"Tumor burden" refers to the total amount of tumor mass distributed throughout the body. Tumor burden refers to the total number of cancer cells or the total size of the tumor throughout the body. Tumor burden can be determined by a variety of methods known in the art, such as measuring the size of a tumor using calipers after the tumor is removed from a subject, or using imaging techniques (e.g., ultrasound, bone scanning, computed tomography (CT), or magnetic resonance imaging (MRI) scanning) when the tumor is *in vivo.*

The term "tumor size" refers to the total size of a tumor, which can be measured as the length and width of the tumor. Tumor size can be determined by a variety of methods known in the art, such as measuring the size of a tumor using calipers after the tumor is removed from a subject, or using imaging techniques (e.g., bone scanning, ultrasound, CT, or MRI scanning) when the tumor is *in vivo.*

The terms "subject" and "individual" include any organism, preferably an animal, more preferably a mammal (such as rat, mouse, dog, cat, rabbit, etc), and most preferably a human. The terms "subject" and "patient" are used interchangeably herein.

The "antibody" described herein refers to any form of antibody that can achieve the desired bioactivity or binding activity. Therefore, it is used in the broadest sense, but is not limited to monoclonal antibodies, polyclonal antibodies, multispecific antibodies, humanized full-length human antibodies, chimeric antibodies, and camelid-derived single-domain antibodies. The "antibody" specifically binds to an antigen and comprises at least two heavy (H) chains and two light (L) chains interconnected by disulfide bonds. Each heavy chain comprises a heavy chain variable region (VH) and a heavy chain constant region, with the heavy chain constant region comprising three constant domains CH1, CH2 and CH3. Each light chain comprises a light chain variable region (VL) and a light chain constant region, with the light chain constant region comprising one constant domain CL. The VH and VL regions can be further divided into hypervariable regions known as complementarity-determining regions (CDRs), which are interspersed among more conserved regions called framework regions (FRs). Generally, both light and heavy chain variable domains comprise FR1, CDR1, FR2, CDR2, FR3, CDR3 and FR4 from N-terminus to C-terminus. Amino acids are generally assigned to each domain according to the following definitions: Sequences of Proteins of Immunological Interest, Kabat et al.; National Institutes of Health, Bethesda, Md.; 5th edition; NIH Publication No. 91-3242 (1991): Kabat (1978) Adv. Prot. Chem. 32:1-75; Kabat et al., (1977) J. Biol. Chem. 252:6609-6616; Chothia et al., (1987) J Mol. Biol. 196:901-917, or Chothia et al., (1989) Nature 341:878-883.

The carboxyl-terminal portion of the heavy chain can define a constant region primarily responsible for effector function. Human light chains are generally classified as κ and λ chains. Human heavy chains are generally classified as µ, δ, γ, α or ε chains, and isotypes of antibodies are defined as IgM, IgD, IgG, IgA, and IgE. The IgG subclass is well known to those skilled in the art and includes, but is not limited to, IgG1, IgG2, IgG3, and IgG4.

The term "antibody" includes: naturally occurring and non-naturally occurring Abs; monoclonal and polyclonal Abs; chimeric and humanized Abs; human or non-human Abs; fully synthetic Abs; and single-chain Abs. Non-human Abs can be humanized by recombinant methods to reduce their immunogenicity in humans.

Unless otherwise specifically indicated, the "antibody fragment" or "antigen-binding fragment" described herein refers to an antigen-binding fragment of an antibody, i.e., an antibody fragment that retains the ability of a full-length antibody to specifically bind to an antigen, e.g., a fragment that retains one or more CDRs. Examples of the antigen-binding fragment include, but are not limited to, Fab, Fab', F(ab')₂ and Fv fragments; a diabody; a linear antibody; a single-chain antibody molecule; and a nanobody and a multispecific antibody formed from antibody fragments.

A "chimeric antibody" refers to an antibody and a fragment thereof in which a portion of the heavy and/or light chain is identical with or homologous to corresponding sequences of an antibody derived from a particular species (e.g., human) or belonging to a particular antibody class or subclass, while the remainder of the chain is identical with or homologous to corresponding sequences of an antibody derived from another species (e.g., mouse) or belonging to another antibody class or subclass, so long as they exhibit the desired bioactivity.

A "human antibody" refers to an antibody that comprises only human immunoglobulin sequences. A human antibody may contain a murine carbohydrate chain if it is produced in mice, mouse cells, or hybridomas derived from mouse cells. Similarly, "mouse antibody" or "rat antibody" refers to an antibody that comprises only mouse or rat immunoglobulin sequences, respectively.

"Humanized antibody" refers to an antibody form containing sequences from both non-human (e.g., murine) and human antibodies. Such antibodies contain minimal sequences derived from non-human immunoglobulins. Typically, a humanized antibody will comprise substantially all of at least one and typically two variable domains, in which all or substantially all of the hypervariable loops correspond to those of a non-human immunoglobulin and all or substantially all of the FRs are those of a human immunoglobulin. The humanized antibody optionally further comprises at least one portion of an immunoglobulin constant region (Fc), typically a human immunoglobulin constant region.

The term "cancer" or "malignant tumor" used herein refers to a wide variety of diseases characterized by the uncontrolled growth of abnormal cells in the body. Unregulated cell division, growth division and growth lead to the formation of malignant tumors that invade adj acent tissues and can also metastasize to distal parts of the body through the lymphatic system or the blood flow. Examples of cancers suitable for treatment or prevention using the method, the medicament and the kit of the present disclosure include, but are not limited to, carcinoma, lymphoma, leukemia, blastoma, and sarcoma. More specific examples of cancer include squamous cell carcinoma, myeloma, small-cell lung cancer, non-small cell lung cancer, glioma, Hodgkin lymphoma, non-Hodgkin lymphoma, acute myeloid leukemia, multiple myeloma, gastrointestinal cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, nasopharyngeal carcinoma, cervical cancer, brain cancer, gastric cancer, bladder cancer, hepatoma, breast cancer, colon cancer, and head and neck cancer.

The term "tumor mutation burden (TMB)" used herein refers to the total number of somatic gene coding errors, base substitutions, and gene insertion or deletion errors detected per million bases. In some embodiments of the present disclosure, tumor mutation burden (TMB) is estimated by analysis of somatic mutations, including coding base substitutions and megabase insertions in the panel sequences studied.

The term "head and neck squamous cell carcinoma (HNSCC)" has its general meaning in the art and refers to tumors occurring in the mouth, nose, pharynx, larynx, and other areas. Generally, head and neck squamous cell carcinoma refers to squamous cell carcinoma of the head and neck. HNSCC may be caused by a variety of factors, including a history of smoking, a history of alcohol consumption, exposure to carcinogens (e.g., areca catechu, air pollutants, etc.), viral infections (e.g., HPV (human papilloma virus) or EBV (epstein-barr virus)), and the like. In some embodiments, the HNSCC is early-stage HNSCC, non-metastatic HNSCC, primary HNSCC, advanced HNSCC, locally advanced HNSCC, metastatic HNSCC, HNSCC in remission, or recurrent HNSCC.

The term "immunotherapy" refers to the treatment of a subject with a disease or at risk of infection or disease recurrence by a method that includes inducing, enhancing, suppressing or otherwise modifying an immune response. The "treatment" or "therapy" of a subject refers to any type of intervention or process performed on the subject, or the administration of an active agent to the subject, with the purpose of reversing, alleviating, ameliorating, slowing or preventing the onset, progression, severity or recurrence of symptoms, complications or conditions, or biochemical indicators associated with the disease.

The term "programmed death receptor-1 (PD-1)" refers to an immunosuppressive receptor belonging to the CD28 family. PD-1 is expressed primarily on previously activated T cells *in vivo* and binds to two ligands, PD-L1 and PD-L2. The term "PD-1" used herein includes human PD-1 (hPD-1), variants, isotypes, and species homologs of hPD-1, and analogs having at least one common epitope with hPD-1.

The term "EGFR" (epidermal growth factor receptor, also known as ERBB1 or HER1) refers to a receptor for signaling such as cell proliferation by epidermal growth factor (EGF), and belongs to the ERBB family (also known as the HER family), which is part of the receptor tyrosine kinase (RTK) family. EGFR is located on the surface of the cell membrane and is activated by binding to ligands, including EGF and TGFα (transforming growth factor α). Upon activation, EGFR transforms from a monomer to a dimer, which activates its intracellular kinase pathways, including MAPK, Akt, JNK, and the like, thereby inducing cell proliferation.

A "therapeutically effective amount" or "therapeutically effective dose" of a medicament or therapeutic agent is any amount of the medicament that, when used alone or in combination with an additional therapeutic agent, protects a subject from the onset of a disease or promotes the regression of a disease as evidenced by a decrease in the severity of disease symptoms, an increase in the frequency and duration of disease asymptomatic period, or the prevention of injury or disability resulting from disease affliction. The ability of a therapeutic agent to promote the regression of a disease can be assessed using a variety of methods known to those skilled in the art, such as in human subjects during clinical trials, in animal model systems that predict human efficacy, or by determining the activity of the agent in an *in vitro* assay.

A therapeutically effective amount of a medicament includes a "prophylactically effective amount" which is any amount of the medicament that, when administered alone or in combination with an anti-tumor agent to a subject at risk of developing cancer or a subject experiencing cancer recurrence, inhibits the development or recurrence of cancer.

A "biotherapeutic agent" refers to a biomolecule, such as an antibody or a fusion protein, that blocks ligand/receptor signaling in any biological pathway that supports tumor maintenance and/or growth or inhibits an anti-tumor immune response.

Unless otherwise specifically indicated, "CDR" used herein refers to a complementarity-determining region of the immunoglobulin variable region defined using the Kabat numbering system.

A "therapeutic anti-PD-1 monoclonal antibody" refers to an antibody that specifically binds to the mature form of specific PD-1 expressed on the surface of certain mammalian cells. Mature PD-1 does not have a secretory leader sequence, also known as a leader peptide. The terms "PD-1" and "mature PD-1" are used interchangeably herein, and unless otherwise specifically defined or clearly seen from the context, they should be understood as referring to the same molecule.

As described herein, a therapeutic anti-human PD-1 antibody or anti-PD-1 antibody refers to a monoclonal antibody that specifically binds to mature human PD-1.

As described herein, a therapeutic anti-human EGFR antibody or anti-EGFR antibody refers to a monoclonal antibody that specifically binds to EGFR.

The "framework region" or "FR" described herein refers to the immunoglobulin variable region excluding CDRs.

An "isolated antibody or antigen-binding fragment thereof" refers to a molecule that is in a purified state, and in this case, is substantially free of other biomolecules, such as nucleic acids, proteins, lipids, carbohydrates, or other materials (such as cell debris or growth medium).

A "patient" or "subject" refers to any single subject in need of a medical procedure or participating in a clinical trial, epidemiological study, or serving as a control, and generally refers to mammals, including humans and other mammals, such as horses, cows, dogs, or cats.

The "RECIST 1.1 efficacy criteria" described herein refer to the definition of target injury or non-target injury described in Eisenhauer, E A et al., Eur. J Cancer 45:228-247 (2009) in the context of the measured response. RECIST 1.1 efficacy criteria are the most commonly used criteria for efficacy evaluation of solid tumors prior to immunotherapy. However, with the advent of the immunotherapy era, many difficulties that were not previously encountered in tumor evaluation have emerged. Therefore, based on the newly emerging phenomenon brought by immunotherapy itself, the RECIST working group revised the existing "RECIST v.1.1" and proposed new judgment criteria in 2016, namely the "irRECIST criteria" described herein, aiming at better evaluating the efficacy of immunotherapeutic drugs.

The term "ECOG" scoring system is an indicator of general health status and tolerance to treatment of patients from their physical strength. The ECOG performance status scoring system includes scores of 0, 1, 2, 3, 4, and 5. A score of 0 means that the motility is completely normal and has no difference from the motility before onset of disease. A score of 1 means that the person is free to walk and can engage in light physical activities, including general housework or office work, but not in heavy physical activities.

A "sustained response" refers to a sustained therapeutic effect following cessation of treatment with a therapeutic agent or combination therapy described herein. In some embodiments, the sustained response has a duration that is at least the same as the duration of treatment or at least 1.5, 2.0, 2.5, or 3 times the duration of the treatment.

A "tissue section" refers to a single portion or piece of a tissue sample, such as a tissue slice cut from a sample of normal tissue or a tumor.

As used herein, "treating" cancer refers to applying a treatment regimen described herein (e.g., administration of an anti-PD-1 antibody) to a subject with or diagnosed with cancer to achieve at least one positive therapeutic effect (e.g., a decrease in cancer cell count, a decrease in tumor volume, a reduction in the rate of cancer cell infiltration into peripheral organs, or a reduction in the rate of tumor metastasis or tumor growth). Positive therapeutic effects in cancer can be measured in a variety of ways (see W. A. Weber, J. Nucl. Med., 50:1S-10S (2009)). For example, T/C ≤ 42% for tumor growth inhibition is the minimum level of anti-tumor activity according to the NCI criteria. It is considered that T/C (%) = median volume of treated tumor/median volume of control tumor × 100. In some embodiments, the therapeutic effect achieved by the combination of the present disclosure is any one of PR, CR, OR, PFS, DFS, and OS. PFS (also called "time to tumor progression") refers to the length of time during and after treatment for which cancer does not grow, and includes the amount of time a patient experiences CR or PR and the amount of time a patient experiences SD. DFS refers to the length of time during and after treatment for which a patient remains disease-free. OS refers to an extension of life expectancy compared to an initially treated or untreated individual or patient. In some embodiments, the response to the combination of the present disclosure is any one of PR, CR, PFS, DFS, OR, or OS, assessed using RECIST 1.1 efficacy criteria. The treatment regimen of the combination of the present disclosure effective in treating a patient with cancer may vary depending upon a variety of factors such as the disease state, age, body weight of the patient, and the ability of the therapy to elicit an anti-cancer response in the subject. Embodiments of the present disclosure may not achieve an effective positive therapeutic effect in each subject, but should be effective and achieve a positive therapeutic effect in a statistically significant number of subjects.

The terms "mode of administration" and "dosing regimen" are used interchangeably and refer to the dose and time of use of each therapeutic agent in the combination of the present disclosure.

The term "immunohistochemistry (IHC)" refers to a method for determining antigens (polypeptides and proteins) in tissue cells by developing chromogenic agents (fluoresceins, enzymes, metal ions, and isotopes) that label antibodies through chemical reactions based on the principle that antigens specifically binds to antibodies, and performing localization, qualitative, and relatively quantitative studies on those antigens. In some embodiments of the present disclosure, a tumor tissue sample from a subject is tested for PD-L1 prior to treatment with an anti-PD-1 antibody by conducting a staining experiment using the anti-human PD-L1 antibody SP142 from Roche (Cat No: M4422). In some embodiments, the presence of tumor cells with membrane staining ≥ 1% is defined as PD-L1 positive.

The term "biosimilar", also referred to as "biogeneric" or "follow-on biologic" or "subsequent entry biologic" refers to a biological article of manufacture that is a substantially identical copy of a product approved by a regulatory agency.

In the following paragraphs, various aspects of the present disclosure are further described in detail.

### Anti-PD-1 antibody

As used herein, an "anti-PD-1 antibody" refers to any chemical compound or biomolecule that binds to PD-1, blocks the binding of PD-L1 expressed on cancer cells to PD-1 expressed on immune cells (T, B, and NK cells), and preferably also blocks the binding of PD-L2 expressed on cancer cells to PD-1 expressed on immune cells. Alternative nouns or synonyms for PD-1 and its ligands include: PDCD1, PD1, CD279, and SLEB2 for PD-1; PDCD1L1, PDL1, B7-H1, B7H1, B7-4, CD274, and B7-H for PD-L1; and PDCD1L2, PDL2, B7-DC, and CD273 for PD-L2. In any of the therapy, medicament, and use described herein for treating a human individual, the anti-PD-1 antibody blocks the binding of human PD-L1 to human PD-1, and preferably blocks the binding of both human PD-L1 and PD-L2 to human PD1. The amino acid sequence of human PD-1 can be found at NCBI locus number: NP_005009. The amino acid sequences of human PD-L1 and PD-L2 can be found at NCBI locus numbers: NP_ 054862 and NP_079515, respectively.

As used herein, unless otherwise indicated or described, when referring to the term "anti-PD-1 antibody", it includes antigen-binding fragments of the antibody.

The anti-PD-1 antibody suitable for use in any of the use, therapy, medicament, and kit described herein has an immunosuppressive effect achieved by binding to PD-1 with high specificity and high affinity, blocking the binding of PD-L1/2 to PD-1, and inhibiting PD-1 signal transduction. In any of the use, therapy, medicament, and kit disclosed herein, the anti-PD-1 antibody includes the full-length antibody itself, as well as an antigen-binding portion or fragment that binds to a PD-1 receptor and exhibits functional properties similar to those of an intact Ab in inhibiting ligand binding and upregulating the immune system. In some embodiments, the anti-PD-1 antibody or the antigen-binding fragment thereof is an anti-PD-1 antibody or an antigen-binding fragment thereof that cross-competes for binding to human PD-1 with toripalimab. In other embodiments, the anti-PD-1 antibody or the antigen-binding fragment thereof is a chimeric, humanized, or human Ab or an antigen-binding fragment thereof. In certain embodiments for treating a human individual, the Ab is a humanized Ab.

In some embodiments, the anti-PD-1 antibody used in any of the use, therapy, medicament, and kit described herein includes a monoclonal antibody (mAb) or an antigen-binding fragment thereof that specifically binds to PD-1, and preferably specifically binds to human PD-1. The mAb may be a human antibody, a humanized antibody, or a chimeric antibody, and may include a human constant region. In some embodiments, the constant region is selected from the group consisting of human IgG1, IgG2, IgG3, and IgG4 constant regions; preferably, the anti-PD-1 antibody or the antigen-binding fragment thereof suitable for use in any of the use, therapy, medicament, and kit described herein comprises a heavy chain constant region of human IgG1 or IgG4 isotype, more preferably a human IgG4 constant region. In some embodiments, the sequence of the IgG4 heavy chain constant region of the anti-PD-1 antibody or the antigen-binding fragment thereof comprises an S228P mutation that replaces a serine residue in a hinge region with a proline residue that is typically present at the corresponding position of an antibody of IgG1 isotype; and the light chain constant region is selected from a light chain constant region of a λ light chain or a κ light chain.

Preferably, in any one of the embodiments of the use, therapy, medicament, and kit described herein, the PD-1 antibody is a monoclonal antibody or an antigen-binding fragment thereof, which comprises light chain CDRs with the amino acids set forth in SEQ ID NOs: 1, 2, and 3, and heavy chain CDRs with the amino acids set forth in SEQ ID NOs: 4, 5, and 6.

More preferably, in any one of the embodiments of the use, therapy, medicament, and kit described herein, the PD-1 antibody is a monoclonal antibody that specifically binds to human PD-1 and comprises: (a) a light chain variable region comprising SEQ ID NO: 7, and (b) a heavy chain variable region comprising SEQ ID NO: 8.

Further preferably, in any one of the embodiments of the use, therapy, medicament, and kit described herein, the PD-1 antibody is a monoclonal antibody that specifically binds to human PD-1 and comprises: (a) a light chain comprising SEQ ID NO: 9, and (b) a heavy chain comprising SEQ ID NO: 10.

Table A below provides the amino acid sequence numbering of the light chain CDRs and heavy chain CDRs of an exemplary anti-PD-1 mAb used in the use, therapy, medicament, and kit described herein:

**Table A: Light and heavy chain CDRs of an exemplary anti-PD-1 antibody**

| | | |
|---|---|---|
| LCDR1 | SEQ ID NO: 1 | RSSQSIVHSNGNTYLE |
| LCDR2 | SEQ ID NO: 2 | KVSNRFS |
| LCDR3 | SEQ ID NO: 3 | FQGSHVPLT |
| HCDR1 | SEQ ID NO: 4 | DYEMH |
| HCDR2 | SEQ ID NO: 5 | VIESETGGTAYNQKFKG |
| HCDR3 | SEQ ID NO: 6 | EGITTVATTYYWYFDV |

SEQ ID NO: 7
SEQ ID NO: 8
SEQ ID NO: 9 
DVVMTQSPLSLPVTLGQPASISCRSSQSIVHSNGNTYLEWYLQKPGQSPQLLIYKVS
NRFSGVPDRFSGSGSGTDFTLKISRVEAEDVGVYYCFQGSHVPLTFGQGTKLEIKRTVAA
PSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
SEQ ID NO: 10

QGQLVQSGAEVKKPGASVKVSCKASGYTFTDYEMHWVRQAPIHGLEWIGVIESET
GGTAYNQKFKGRVTITADKSTSTAYMELSSLRSEDTAVYYCAREGITTVATTYYWYFD
VWGQGTTVTVSSASTKGPSVFPLAPCSRSTSESTAALGCLVKDYFPEPVTVSWNSGALT
SGVHTFPAVLQSSGLYSLSSVVTVPSSSLGTKTYTCNVDHKPSNTKVDKRVESKYGPPCP

PCPAPEFLGGPSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSDGSF
FLYSRLTVDKSRWQEGNVFSCSVMHEALHNHYTQKSLSLSLGK

An example of an anti-PD-1 antibody that binds to human PD-1 and can be used in the use, therapy, medicament, and kit described herein is described in WO2014206107. The human PD-1 mAb that can be used as the anti-PD-1 antibody in the use, therapy, medicament, and kit described herein includes any one of the anti-PD-1 antibodies described in WO2014206107, including toripalimab (a humanized IgG4 mAb having the structure described in WHO Drug Information, 32(2):372-373 (2018) and comprising the light and heavy chain amino acid sequences set forth in SEQ ID NOs: 9 and 10). In a preferred embodiment, the anti-PD-1 antibody that can be used in any one of the use, therapy, medicament, and kit described herein is selected from the humanized antibodies 38, 39, 41, and 48 described in WO2014206107. In a particularly preferred embodiment, the anti-PD-1 antibody that can be used in any one of the use, therapy, medicament, and kit described herein is toripalimab.

The anti-PD-1 antibody that can be used in any one of the use, therapy, medicament, and kit described herein also includes nivolumab and pembrolizumab that have been approved by the FDA.

In certain embodiments, the anti-PD-1 antibody that can be used in any one of the use, therapy, medicament, and kit described herein also includes an anti-PD-L1 monoclonal antibody that specifically binds to PD-L1 to block the binding of PD-L1 to PD-1, such as nivolumab or a biosimilar thereof, pembrolizumab or a biosimilar thereof, toripalimab or a biosimilar thereof, sintilimab or a biosimilar thereof, camrelizumab or a biosimilar thereof, tislelizumab or a biosimilar thereof, cemiplimab or a biosimilar thereof, zimberelimab or a biosimilar thereof, penpulimab or a biosimilar thereof, and serplulimab or a biosimilar thereof.

"PD-L1" expression or "PD-L2" expression described herein refers to any detectable expression level of a specific PD-L protein on the surface of a cell or a specific PD-L mRNA within a cell or tissue. PD-L protein expression can be detected in IHC analysis of tumor tissue sections or by flow cytometry using diagnostic PD-L antibodies. Alternatively, PD-L protein expression of tumor cells can be detected by PET imaging using a binding agent that specifically binds to a desired PD-L target (e.g., PD-L1 or PD-L2).

Methods for quantifying PD-L1 protein expression in IHC analysis of tumor tissue sections are found in, but are not limited to, Thompson, R. H. et al., PNAS 101(49):17174-17179 (2004); Taube, J. M. et al., Sci Transl Med 4, 127ra37 (2012); and Toplian, S. L. et al., New Eng. J. Med. 366(26): 2443-2454 (2012), and the like.

In one method, a simple binary endpoint of positive or negative PD-L1 expression is adopted, where the positive expression is defined by the percentage of tumor cells showing histological evidence of cell surface membrane staining. The case where tumor cells on a tumor tissue section account for at least 1% of the total tumor cells is defined as positive PD-L1 expression.

In another method, PD-L1 expression in the tumor tissue section is quantified in tumor cells as well as in infiltrating immune cells. The percentage of tumor cells and infiltrating immune cells exhibiting membrane staining are quantified individually as < 1%, 1% to 50%, and subsequent 50% to 100%. For tumor cells, the PD-L1 expression is counted as negative if the score is < 1%, and positive if the score is ≥ 1%.

In some embodiments, the expression level of PD-L1 by malignant cells and/or by infiltrating immune cells within the tumor is determined to be "overexpressed" or "elevated" based on comparison with the expression level of PD-L1 by an appropriate control. For example, the protein or mRNA expression level of control PD-L1 can be a quantified level in non-malignant cells of the same type or in sections from matched normal tissue.

### Anti-EGFR antibody

As used herein, an "anti-EGFR antibody" refers to any chemical compound or biomolecule that binds to EGFR and inhibits the binding of EGF to EGFR. The EGFR is also referred to as ERBB1 or HER1 with a Gene ID number of 1956, and the corresponding protein numbers include NP_001333826.1, NP_001333827.1, NP_001333828.1, NP_001333829.1, NP_001333870.1, NP_005219.2, NP_958439.1, NP_958440.1, and NP_958441.1.

As used herein, unless otherwise indicated or described, when referring to the term "anti-EGFR antibody", it includes antigen-binding fragments of the antibody.

The anti-EGFR antibody suitable for use in any of the use, therapy, medicament, and kit described herein has an effect of inhibiting tumor angiogenesis, growth, and metastasis achieved by binding to EGFR with high specificity and high affinity, blocking the binding of EGFR to EGF, and thus inhibiting the biological effects of EGFR, including affecting vascular permeability, proliferation, and endothelial cell migration and survival. In any of the use, therapy, medicament, and kit disclosed herein, the anti-EGFR antibody includes the full-length antibody itself, as well as an antigen-binding portion or fragment that binds to an EGFR receptor and exhibits functional properties similar to those of an intact Ab in inhibiting ligand binding and inhibiting vascular endothelial cell proliferation and activation. In some embodiments, the anti-EGFR antibody or the antigen-binding fragment thereof is an anti-EGFR antibody or an antigen-binding fragment thereof that cross-competes for binding to human EGFR with cetuximab. In other embodiments, the anti-EGFR antibody or the antigen-binding fragment thereof is a chimeric, humanized, or human Ab or an antigen-binding fragment thereof. In certain embodiments for treating a human individual, the Ab is a humanized Ab.

In some embodiments, the anti-EGFR antibody used in any of the use, therapy, medicament, and kit described herein includes a monoclonal antibody (mAb) or an antigen-binding fragment thereof that specifically binds to EGFR, and preferably specifically binds to human EGFR. The mAb may be a human antibody, a humanized antibody, or a chimeric antibody, and may include a human constant region. In some embodiments, the heavy chain constant region is selected from the group consisting of human IgG1, IgG2, IgG3, and IgG4 heavy chain constant regions; preferably, the anti-EGFR antibody or the antigen-binding fragment thereof suitable for use in any of the use, therapy, medicament, and kit described herein comprises a heavy chain constant region of human IgG1 or IgG4 isotype, more preferably a human IgG1 heavy chain constant region; and the light chain constant region is selected from a light chain constant region of a λ light chain or a κ light chain, preferably a κ light chain.

Preferably, in any one of the embodiments of the use, therapy, medicament, and kit described herein, the anti-EGFR antibody is a monoclonal antibody or an antigen-binding fragment thereof, which comprises light chain CDRs with the amino acids set forth in SEQ ID NOs: 11, 12, and 13, and heavy chain CDRs with the amino acids set forth in SEQ ID NOs: 14, 15, and 16.

More preferably, in any one of the embodiments of the use, therapy, medicament, and kit described herein, the EGFR antibody is a monoclonal antibody that specifically binds to human EGFR and comprises: (a) a light chain variable region comprising SEQ ID NO: 17, and (b) a heavy chain variable region comprising SEQ ID NO: 18.

Further preferably, in any one of the embodiments of the use, therapy, medicament, and kit described herein, the EGFR antibody is a monoclonal antibody that specifically binds to human EGFR and comprises: (a) a light chain comprising SEQ ID NO: 19, and (b) a heavy chain comprising SEQ ID NO: 20.

Table B below provides the amino acid sequence numbering of the light chain CDRs and heavy chain CDRs of an exemplary anti-EGFR mAb used in the use, therapy, medicament, and kit described herein:

**Table B: Light and heavy chain CDRs of an exemplary anti-EGFR antibody**

| | | |
|---|---|---|
| LCDR1 | SEQ ID NO: 11 | RASQSIGTNIH |
| LCDR2 | SEQ ID NO: 12 | YASESIS |
| LCDR3 | SEQ ID NO: 13 | QQNNNWPTT |
| HCDR1 | SEQ ID NO: 14 | NYGVH |
| HCDR2 | SEQ ID NO: 15 | VIWSGGNTDYNTPFTS |
| HCDR3 | SEQ ID NO: 16 | ALTYYDYEFAY |

SEQ ID NO: 17 
DILLTQSPVILSVSPGERVSFSCRASQSIGTNIHWYQQRTNGSPRLLIKYASESISGIPS
RFSGSGSGTDFTLSINSVESEDIADYYCQQNNNWPTTFGAGTKLELK
 SEQ ID NO: 18 
QVQLKQSGPGLVQPSQSLSITCTVSGFSLTNYGVHWVRQSPGKGLEWLGVIWSGG
NTDYNTPFTSRLSINKDNSKSQVFFKMNSLQSNDTAIYYCARALTYYDYEFAYWGQGT
LVTVSA
SEQ ID NO: 19 
DILLTQSPVILSVSPGERVSFSCRASQSIGTNIHWYQQRTNGSPRLLIKYASESISGIPS
RFSGSGSGTDFTLSINSVESEDIADYYCQQNNNWPTTFGAGTKLELKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC
SEQ ID NO: 20

QVQLKQSGPGLVQPSQSLSITCTVSGFSLTNYGVHWVRQSPGKGLEWLGVIWSGG
NTDYNTPFTSRLSINKDNSKSQVFFKMNSLQSNDTAIYYCARALTYYDYEFAYWGQGT
LVTVSAASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPA
PELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

The human EGFR mAb that can be used as the anti-EGFR antibody in the use, therapy, medicament, and kit described herein includes cetuximab, which is a humanized IgG1 mAb comprising the light chain and heavy chain amino acid sequences set forth in SEQ ID NOs: 19 and 20. In a preferred embodiment, the anti-EGFR antibody that can be used in any one of the use, therapy, medicament, and kit described herein is selected from one or more of necitumumab or a biosimilar thereof, nimotuzumab or a biosimilar thereof, panitumumab or a biosimilar thereof, and cetuximab or a biosimilar thereof. In a particularly preferred embodiment, the anti-EGFR antibody that can be used in any one of the use, therapy, medicament, and kit described herein is cetuximab.

The anti-EGFR antibody that can be used in any one of the use, therapy, medicament, and kit described herein also includes necitumumab or a biosimilar thereof, nimotuzumab or a biosimilar thereof, panitumumab or a biosimilar thereof, and cetuximab or a biosimilar thereof.

### Pharmaceutical combination

The present disclosure provides a pharmaceutical combination comprising an anti-PD-1 antibody or an antigen-binding fragment thereof and an anti-EGFR antibody or an antigen-binding fragment thereof. In the pharmaceutical combination, the anti-PD-1 antibody and the anti-EGFR antibody may be provided in a mixture of the two (i.e., in a form of a pharmaceutical composition), or each may be provided in a separate formulation. In some embodiments, each pharmaceutical combination comprises 1 dose of the anti-PD-1 antibody or the antigen-binding fragment thereof described herein and 3 doses of the anti-EGFR antibody or the antigen-binding fragment thereof described herein, wherein the anti-PD-1 antibody or the antigen-binding fragment thereof and the anti-EGFR antibody or the antigen-binding fragment thereof are provided in separate formulations.

In some embodiments, the anti-PD-1 antibody described herein may be as described in any one of the embodiments herein, more preferably an antibody comprising light chain CDRs with the amino acids set forth in SEQ ID NOs: 1, 2, and 3 and heavy chain CDRs with the amino acids set forth in SEQ ID NOs: 4, 5, and 6, more preferably a monoclonal antibody comprising the light chain variable region set forth in SEQ ID NO: 7 and the heavy chain variable region set forth in SEQ ID NO: 8, more preferably a monoclonal antibody comprising the light chain set forth in SEQ ID NO: 9 and the heavy chain set forth in SEQ ID NO: 10, more preferably the humanized antibodies 38, 39, 41, and 48 described in WO2014206107, and most preferably toripalimab or a biosimilar thereof.

In some embodiments, the anti-EGFR antibody described herein may be as described in any one of the embodiments herein, more preferably an antibody comprising light chain CDRs with the amino acids set forth in SEQ ID NOs: 11, 12, and 13 and heavy chain CDRs with the amino acids set forth in SEQ ID NOs: 14, 15, and 16, more preferably a monoclonal antibody comprising the light chain variable region set forth in SEQ ID NO: 17 and the heavy chain variable region set forth in SEQ ID NO: 18, more preferably a monoclonal antibody comprising the light chain set forth in SEQ ID NO: 19 and the heavy chain set forth in SEQ ID NO: 20, and more preferably cetuximab or a biosimilar thereof.

As described herein, a "pharmaceutically acceptable carrier" includes any and all solvents, dispersion media, coatings, antibacterial and antifungal agents, isotonic and absorption delaying agents, and the like that are physiologically compatible. Preferably, the carrier suitable for use in the composition comprising the anti-PD-1 antibody or the anti-EGFR antibody is suitable for intravenous, intramuscular, subcutaneous, parenteral, spinal, or epidermal administration. The pharmaceutical composition of the present disclosure may comprise one or more pharmaceutically acceptable salts, antioxidants, water, non-aqueous carriers, and/or adjuvants such as stabilizers, preserving agents, wetting agents, emulsifying agents, and dispersing agents.

The content of the anti-cancer active ingredients (e.g., the anti-PD-1 antibody and the anti-EGFR antibody as described herein) in each dose of the drug of the present disclosure is typically the amount of each of these anti-cancer active ingredients administered in a single dose. For example, for a fixed dose of 240 mg per administration of the anti-PD-1 antibody described herein, each dose of the drug may comprise 240 mg of the anti-PD-1 antibody. Of course, for example, for oral tablets, the 240 mg of the anti-PD-1 antibody may be divided into 2 or more tablets, as long as all of these tablets are taken at the time of administration to achieve an administration dose of 240 mg. For the anti-EGFR antibody administered at an individual body surface area dose of about 250 mg/m² individual body surface area, the content of the anti-EGFR antibody in each dose of the drug should be sufficient for a single administration at a dose of 250 mg/m² individual body surface area.

The pharmaceutical combination herein comprises the anti-PD-1 antibody or the antigen-binding fragment thereof and the anti-EGFR antibody or the antigen-binding fragment thereof in amounts sufficient for administration according to the treatment or prevention methods described herein. Specifically, the pharmaceutical combination described herein comprises the anti-PD-1 antibody or the antigen-binding fragment thereof in an amount sufficient for administration at a frequency of about once every week, once every two weeks, once every three weeks, once every four weeks, or once a month, preferably once every three weeks, and the anti-EGFR antibody or the antigen-binding fragment thereof in an amount sufficient for administration at a frequency of about once every week, once every two weeks, once every three weeks, once every four weeks, or once a month, preferably once every week. Preferably, in the pharmaceutical combination described herein, the amount of the anti-PD-1 antibody or the antigen-binding fragment thereof is sufficient for administration at the following single doses: about 0.1 mg/kg individual body weight to about 10.0 mg/kg individual body weight, preferably about 1.0 mg/kg individual body weight to about 10.0 mg/kg individual body weight, such as about 0.1 mg/kg individual body weight, about 0.3 mg/kg individual body weight, about 1 mg/kg individual body weight, about 2 mg/kg individual body weight, about 3 mg/kg individual body weight, about 5 mg/kg individual body weight, or about 10 mg/kg individual body weight, or a fixed dose of about 120 mg to about 480 mg, preferably a fixed dose of about 120 mg to about 360 mg, such as a fixed dose of about 120 mg, about 240 mg, about 360 mg, or about 480 mg, preferably a fixed dose of about 240 mg; and the amount of the anti-EGFR antibody or the antigen-binding fragment thereof is sufficient for administration at the following single doses: about 100 mg/m² individual body surface area to about 500 mg/m² individual body surface area, preferably 200 mg/m² individual body surface area to about 500 mg/m² individual body surface area, such as about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area, preferably about 200 mg/m² individual body surface area, about 250 mg/m² individual body surface area, about 300 mg/m² individual body surface area, about 350 mg/m² individual body surface area, or about 400 mg/m² individual body surface area.

In some embodiments, the pharmaceutical combination comprises 1 dose of the anti-PD-1 antibody or the antigen-binding fragment thereof described herein and 3 doses of the anti-EGFR antibody or the antigen-binding fragment thereof described herein, wherein the anti-PD-1 antibody or the antigen-binding fragment thereof and the anti-EGFR antibody or the antigen-binding fragment thereof are provided in separate formulations, wherein the 1 dose of the formulation of the anti-PD-1 antibody or the antigen-binding fragment thereof comprises 240 mg of the anti-PD-1 antibody or the antigen-binding fragment thereof; among the 3 doses of the formulation of the anti-EGFR antibody or the antigen-binding fragment thereof, 1 dose of the formulation comprises the anti-EGFR antibody or the antigen-binding fragment thereof sufficient for administration at a daily dose of 400 mg/m² individual body surface area, while the other 2 doses are sufficient for administration at a daily dose of 250 mg/m² individual body surface area. In some other embodiments, each of the 3 doses of the formulation of the anti-EGFR antibody or the antigen-binding fragment thereof comprises the anti-EGFR antibody or the antigen-binding fragment thereof sufficient for administration at a daily dose of 250 mg/m² individual body surface area. Preferably, the anti-PD-1 antibody is toripalimab, and the anti-EGFR antibody is cetuximab.

### Dose and dosing regimen

The selection of a dosing regimen (also referred to herein as an administration regimen) for the pharmaceutical combination of the present disclosure depends on several factors, including the solid serum or tissue turnover rate of a treated individual, the level of symptoms, overall immunogenicity, and the degree of accessibility of the target cell, tissue or organ. Preferably, the dosing regimen maximizes the amount of each therapeutic agent delivered to a patient, consistent with an acceptable level of side effects. Thus, the dose and frequency of administration of each of biotherapeutic and chemotherapeutic agents will depend, in part, on the specific therapeutic agent, the severity of the cancer being treated, and the characteristics of the patient. Guidance on selecting appropriate doses of antibodies, cytokines, and small molecules is available. See, for example, Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, NY; Bach (ed.) (1993) Monoclonal Antibodies and Peptide Therapy in Autoimmune Diseases, Marcel Dekker, New York, NY; Baert et al., (2003) New Engl. J. Med. 348:601-608; Milgrom et al., (1999) New Engl. J. Med. 341:1966-1973; Slamon et al., (2001) New Engl. J. Med. 344:783-792; Beniaminovitz et al., (2000) New Engl. J. Med. 342:613-619; Ghosh et al., (2003) New Engl. J. Med. 348:24-32; Lipsky et al., (2000) New Engl. J. Med. 343:1594-1602; Physicians' Desk Reference 2003 (Physicians' Desk Reference, 57th Ed); Medical Economics Company; ISBN: 1563634457; 57th edition (November, 2002). Determination of an appropriate dose regimen can be made by a clinician, for example, with reference to parameters or factors known or suspected to affect treatment or expected to affect treatment in the art, and will depend on, for example, the clinical history of the patient (e.g., previous treatments), the type and stage of the cancer being treated, and biomarkers responsive to one or more therapeutic agents in the combination therapy.

Each therapeutic agent of the pharmaceutical combination of the present disclosure may be administered simultaneously (i.e., in the same pharmaceutical composition), concurrently (i.e., in separate pharmaceutical formulations, one after the other in any order), or sequentially in any order. Sequential administration is particularly useful where the therapeutic agents in the pharmaceutical combination can be in different dosage forms (one drug is a tablet or capsule and the other drug is a sterile liquid formulation) and/or on different dosing schedules (e.g., the chemotherapeutic agent is administered at least daily and the biotherapeutic agent is administered less frequently (e.g., once every week, once every two weeks, or once every three weeks)).

In some embodiments, at least one of the therapeutic agents in the pharmaceutical combination is administered using the same dose regimen (therapeutic dose, frequency, and duration) as that generally used when the agent is used as a monotherapy for treating the same tumor. In other embodiments, the patient receives at least one of the therapeutic agents in the combination therapy at a lower total amount than that when the agent is used as a monotherapy, such as a lower dose, a lower dosing frequency, and/or a shorter duration of treatment.

Each therapeutic agent in the pharmaceutical combination of the present disclosure can be administered orally or parenterally, including intravenous, intramuscular, intraperitoneal, subcutaneous, rectal, topical, and transdermal routes of administration.

The anti-PD-1 antibody of the present disclosure can be administered by continuous infusion or by intermittent dosing, and the single dose may be in a range of about 0.01 mg/kg individual body weight to about 20.0 mg/kg individual body weight, about 0.1 mg/kg individual body weight to about 10.0 mg/kg individual body weight, about 1.0 mg/kg individual body weight to about 10.0 mg/kg individual body weight, or at a fixed dose of about 120 mg to about 480 mg, or at a fixed dose of about 120 mg to about 360 mg. For example, the dose may be about 0.1 mg/kg individual body weight, about 0.3 mg/kg individual body weight, about 1 mg/kg individual body weight, about 2 mg/kg individual body weight, about 3 mg/kg individual body weight, about 5 mg/kg individual body weight, or about 10 mg/kg individual body weight, or a fixed dose of about 120 mg, about 240 mg, about 360 mg, or about 480 mg. The dosing regimen is generally designed to achieve an exposure that results in sustained receptor occupancy (RO) based on the typical pharmacokinetics of Ab. A representative dosing regimen may be about once every week, about once every two weeks, about once every three weeks, about once every four weeks, about once a month, or longer. In some embodiments, the anti-PD-1 antibody is administered to an individual about once every three weeks.

In some embodiments, the anti-PD-1 antibody of the present disclosure is toripalimab administered at a single dose selected from about 1.0 mg/kg individual body weight to about 10.0 mg/kg individual body weight, a fixed dose of about 120 mg to about 480 mg, or a fixed dose of about 120 mg to about 360 mg. In some embodiments, toripalimab is administered intravenously at a single dose selected from about 1 mg/kg individual body weight, about 2 mg/kg individual body weight, about 3 mg/kg individual body weight, about 4 mg/kg individual body weight, and about 5 mg/kg individual body weight, or fixed doses of about 120 mg, about 240 mg, and about 360 mg. In some preferred embodiments, toripalimab is administered as a liquid drug at a selected dose by intravenous infusion over a period of at least 30 min, such as about 60 min. In some embodiments, toripalimab is administered at a dose of about 3 mg/kg individual body weight or a fixed dose of about 240 mg by intravenous infusion over a period of at least 30 min, such as about 60 min, once every three weeks (Q3W).

The anti-EGFR antibody of the present disclosure can be administered by continuous infusion or by intermittent dosing, and the single dose may be in a range of about 100 mg/m² individual body surface area to about 500 mg/m² individual body surface area, preferably 200 mg/m² individual body surface area to about 500 mg/m² individual body surface area, such as about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area, preferably about 200 mg/m² individual body surface area, about 250 mg/m² individual body surface area, about 300 mg/m² individual body surface area, about 350 mg/m² individual body surface area, or about 400 mg/m² individual body surface area. The dosing regimen is generally designed to achieve an exposure that results in sustained receptor occupancy (RO) based on the typical pharmacokinetics of Ab. A representative dosing regimen may be about once every week, about once every two weeks, about once every three weeks, about once every four weeks, about once a month, or longer. In some embodiments, the anti-EGFR antibody is administered to an individual about once every week. In some embodiments, the anti-EGFR antibody is first administered at a dose of 300 mg/m² individual body surface area or more, followed by administration at a dose of 250 mg/m² individual body surface area or more.

In some embodiments, the anti-EGFR antibody of the present disclosure is cetuximab administered at a single dose selected from about 100 mg/m² individual body surface area to about 500 mg/m² individual body surface area, or about 200 mg/m² individual body surface area to about 500 mg/m² individual body surface area. In some embodiments, cetuximab is administered intravenously at a single dose selected from a dose of about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area. In some preferred embodiments, cetuximab is administered as a liquid drug at a selected dose by intravenous infusion over a period of at least 15 min, such as about 30 min. In some embodiments, cetuximab is administered at a dose of about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area by intravenous infusion over a period of at least 15 min, such as about 30 min, once every week (QW). In some embodiments, cetuximab is first administered at a dose of 300 mg/m² individual body surface area or more (e.g., at a first dose of 400 mg/m² individual body surface area), followed by each subsequent administration at a dose of 250 mg/m² individual body surface area or more (e.g., followed by administration at a dose of 250 mg/m² individual body surface area or 200 mg/m² individual body surface area).

In some embodiments, toripalimab is administered alone.

In some embodiments, in each administration cycle, toripalimab is first administered followed by administration of cetuximab, and the two can be administered on the same day or with an interval of up to 3 days.

In some embodiments, toripalimab is administered at a fixed dose of about 240 mg, Q3W, and cetuximab is administered at a dose of about 250 mg/m² individual body surface area or about 400 mg/m² individual body surface area, QW.

The administration cycles for the anti-PD-1 antibody and the anti-EGFR antibody of the present disclosure may be identical or different, and may be one week, two weeks, three weeks, one month, two months, three months, four months, five months, half a year, one year, two years, or longer; optionally, the administration cycles may have identical or different durations, and may be at identical or different intervals. For example, in some embodiments, toripalimab is administered at a fixed dose of about 240 mg once every three weeks, and cetuximab is administered at a dose of about 250 mg/m² individual body surface area or about 400 mg/m² individual body surface area once every week.

In some embodiments, toripalimab is administered at a fixed dose of about 240 mg once every three weeks, and cetuximab is first administered at a dose of about 400 mg/m² individual body surface area, followed by each subsequent administration at a dose of about 250 mg/m² individual body surface area once every week; preferably, cetuximab is administered at least about 60 min after administration of toripalimab.

### Treatment method and use

The present disclosure relates to use of an anti-PD-1 antibody or an antigen-binding fragment thereof in the preparation of a medicament or kit for preventing or treating head and neck squamous cell carcinoma (HNSCC), or use of a combination of an anti-PD-1 antibody or an antigen-binding fragment thereof and an anti-EGFR antibody or an antigen-binding fragment thereof in the preparation of a medicament or kit for preventing or treating head and neck squamous cell carcinoma. Preferably, the combination is the pharmaceutical combination as described in any one of the embodiments herein.

The present disclosure further relates to a method for preventing or treating head and neck squamous cell carcinoma, comprising administering to an individual in need an effective amount of an anti-PD-1 antibody or an antigen-binding fragment thereof, or a combination of an anti-PD-1 antibody or an antigen-binding fragment thereof and an anti-EGFR antibody or an antigen-binding fragment thereof. Preferably, the anti-PD-1 antibody or the antigen-binding fragment thereof and the anti-EGFR antibody or the antigen-binding fragment thereof are administered at the doses and dosing regimens as described in any one of the embodiments herein.

The present disclosure further relates to an anti-PD-1 antibody or an antigen-binding fragment thereof, or a combination of an anti-PD-1 antibody or an antigen-binding fragment thereof and an anti-EGFR antibody or an antigen-binding fragment thereof, for use in the prevention or treatment of head and neck squamous cell carcinoma. Preferably, the combination is the pharmaceutical combination as described in any one of the embodiments herein.

The head and neck squamous cell carcinoma may be as described in any one of the preceding embodiments; preferably, the head and neck squamous cell carcinoma is recurrent or metastatic head and neck squamous cell carcinoma, preferably recurrent or metastatic head and neck squamous cell carcinoma that has failed a previous first-line platinum-based chemotherapy regimen. Further, the head and neck squamous cell carcinoma has ≥ 1 measurable lesion according to RECIST v1.1 criteria.

Preferably, the anti-PD-1 antibody used for head and neck squamous cell carcinoma may be as described in any one of the embodiments herein, more preferably an antibody comprising light chain CDRs with the amino acids set forth in SEQ ID NOs: 1, 2, and 3 and heavy chain CDRs with the amino acids set forth in SEQ ID NOs: 4, 5, and 6, more preferably a monoclonal antibody comprising the light chain variable region set forth in SEQ ID NO: 7 and the heavy chain variable region set forth in SEQ ID NO: 8, more preferably a monoclonal antibody comprising the light chain set forth in SEQ ID NO: 9 and the heavy chain set forth in SEQ ID NO: 10, more preferably the humanized antibodies 38, 39, 41, and 48 described in WO2014206107, and most preferably toripalimab or a biosimilar thereof.

Preferably, the anti-EGFR antibody used for head and neck squamous cell carcinoma may be as described in any one of the embodiments herein, more preferably an antibody comprising light chain CDRs with the amino acids set forth in SEQ ID NOs: 11, 12, and 13 and heavy chain CDRs with the amino acids set forth in SEQ ID NOs: 14, 15, and 16, more preferably a monoclonal antibody comprising the light chain variable region set forth in SEQ ID NO: 17 and the heavy chain variable region set forth in SEQ ID NO: 18, more preferably a monoclonal antibody comprising the light chain set forth in SEQ ID NO: 19 and the heavy chain set forth in SEQ ID NO: 20, and preferably cetuximab or a biosimilar thereof.

Preferably, the anti-PD-1 antibody used for head and neck squamous cell carcinoma is toripalimab or a biosimilar thereof, and the anti-EGFR antibody used for head and neck squamous cell carcinoma is cetuximab or a biosimilar thereof.

More preferably, the anti-PD-1 antibody used for head and neck squamous cell carcinoma is toripalimab, and the anti-EGFR antibody used for head and neck squamous cell carcinoma is cetuximab.

### Kit

The present disclosure further provides a kit comprising one or more single dosage units of an anti-PD-1 antibody or an antigen-binding fragment thereof, wherein preferably, the anti-PD-1 antibody or the antigen-binding fragment thereof is as described in any one of the embodiments herein, and more preferably, the anti-PD-1 antibody is toripalimab or a biosimilar thereof.

In some embodiments, the kit of the present disclosure comprises one or more single dosage units of an anti-PD-1 antibody or an antigen-binding fragment thereof and one or more single dosage units of an anti-EGFR antibody or an antigen-binding fragment thereof, wherein preferably, the anti-PD-1 antibody or the antigen-binding fragment thereof is as described in any one of the embodiments herein, and preferably, the anti-EGFR antibody or the antigen-binding fragment thereof is as described in any one of the embodiments herein.

In some embodiments, the kit of the present disclosure comprises toripalimab or a biosimilar thereof and cetuximab or a biosimilar thereof as an anti-cancer active formulation in the kit.

The anti-cancer active ingredients in the kit may be provided independently. For example, the kit may comprise one or more single dosage units of an anti-PD-1 antibody or an antigen-binding fragment thereof (preferably toripalimab), and an anti-EGFR antibody or an antigen-binding fragment thereof (preferably cetuximab). Preferably, the single dosage unit comprises the anti-PD-1 antibody or the antigen-binding fragment thereof in an amount of a fixed dose of about 120 mg to about 480 mg, such as about 120 mg, about 240 mg, about 360 mg, or about 480 mg, preferably a dose of about 240 mg; and the single dosage unit comprises the anti-EGFR antibody or the antigen-binding fragment thereof in an amount sufficient for administration to the patient at a single dose of about 100 mg/m² individual body surface area to about 500 mg/m² individual body surface area, preferably 200 mg/m² individual body surface area to about 500 mg/m² individual body surface area, such as about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area, preferably about 200 mg/m² individual body surface area, about 250 mg/m² individual body surface area, about 300 mg/m² individual body surface area, about 350 mg/m² individual body surface area, or about 400 mg/m² individual body surface area.

In some embodiments, the kit of the present disclosure comprises the pharmaceutical combination as described in any one of the embodiments herein, wherein the pharmaceutical combination comprises 1 dose of the anti-PD-1 antibody (preferably toripalimab) or the antigen-binding fragment thereof and 3 doses of the anti-EGFR antibody (preferably cetuximab) or the antigen-binding fragment thereof, wherein the 1 dose of the formulation of the anti-PD-1 antibody or the antigen-binding fragment thereof comprises 240 mg of the anti-PD-1 antibody or the antigen-binding fragment thereof; among the 3 doses of the formulation of the anti-EGFR antibody or the antigen-binding fragment thereof, 1 dose of the formulation comprises the anti-EGFR antibody or the antigen-binding fragment thereof sufficient for administration at a daily dose of 400 mg/m² individual body surface area, while the other 2 doses are sufficient for administration at a daily dose of 250 mg/m² individual body surface area, or each of the 3 doses of the formulation of the anti-EGFR antibody or the antigen-binding fragment thereof comprises the anti-EGFR antibody or the antigen-binding fragment thereof sufficient for administration at a daily dose of 250 mg/m² individual body surface area.

In some embodiments, the pharmaceutical combination comprises 2 or more pharmaceutical combinations as described in any one of the embodiments herein, wherein each of the pharmaceutical combinations comprises 1 dose of the anti-PD-1 antibody described herein (preferably, toripalimab) or the antigen-binding fragment thereof and 3 doses of the anti-EGFR antibody described herein (preferably, cetuximab) or the antigen-binding fragment thereof, and the anti-PD-1 antibody or the antigen-binding fragment thereof and the anti-EGFR antibody or the antigen-binding fragment thereof are provided in separate formulations, wherein in 1 of the combinations, the 1 dose of the formulation of the anti-PD-1 antibody or the antigen-binding fragment thereof comprises 240 mg of the anti-PD-1 antibody or the antigen-binding fragment thereof, and among the 3 doses of the formulation of the anti-EGFR antibody or the antigen-binding fragment thereof, 1 dose of the formulation comprises the anti-EGFR antibody or the antigen-binding fragment thereof sufficient for administration at a daily dose of 400 mg/m² individual body surface area, while the other 2 doses are sufficient for administration at a daily dose of 250 mg/m² individual body surface area; in the remaining combinations, each of the 3 doses of the formulation of the anti-EGFR antibody or the antigen-binding fragment thereof comprises the anti-EGFR antibody or the antigen-binding fragment thereof sufficient for administration at a daily dose of 250 mg/m² individual body surface area.

### Abbreviation

The following abbreviations are used throughout the specification and examples of the present disclosure:
- CDR: Complementarity-determining region
- DFS: Disease-free survival
- DOR: Duration of response
- ECOG: Eastern cooperative oncology group
- FR: Framework region
- IgG: Immunoglobulin G
- IHC: Immunohistochemistry
- OR: Overall response
- ORR: Objective response rate
- OS: Overall survival
- mOS: Mean overall survival
- PD: Progressive disease
- PFS: Progression-free survival
- mPFS: Mean progression-free survival
- PR: Partial response
- CR: Complete response
- SD: Stable disease
- DLT: Dose-limiting toxicity
- MTD: Maximum tolerated dose
- AE: Adverse event
- QW: One dose every week
- Q3W: One dose every three weeks
- QD: One dose every day
- IRC: Independent Review Committee
- TRAE: Treatment-related adverse event
- irAE: Immune-related adverse event
- SAE: Serious adverse event
- RO: Receptor occupancy
- HNSCC: Head and neck squamous cell carcinoma
- RECIST: Response Evaluation Criteria in Solid Tumors
- iRECIST: Modified Immune-Related Response Evaluation Criteria in Solid Tumors
- irRECIST: Immune-Related Response Evaluation Criteria in Solid Tumors
- mRECIST: Modified Response Evaluation Criteria in Solid Tumors
- imRECIST: Immune-Modified Response Evaluation Criteria in Solid Tumors
- DOR: Duration of response
- MSI: Microsatellite instability
- CI: Confidence interval
- DCR: Disease control rate
- TTP: Time to progression
- HNSCC: Head and neck squamous cell carcinoma
- CPS: Combined positive score

The present disclosure is further illustrated by the following examples, which should not be construed as limiting the present disclosure. The contents of all references cited throughout the present application are explicitly incorporated herein by reference.

### Examples

### Example 1: Anti-PD-1 Antibody in Combination with Anti-EGFR Antibody for Treatment of Recurrent or Metastatic HNSCC That Had Failed First-Line Platinum-Based Chemotherapy Regimen

### 1.1 Study design

This study is an open, multicenter Phase Ib/II clinical study. The Phase Ib study primarily aimed to evaluate the safety of toripalimab in combination with cetuximab in the treatment of recurrent or metastatic HNSCC that had failed a first-line platinum-based chemotherapy regimen, and to determine the recommended Phase II dose (RP2D). The Phase II study was divided into two cohorts. Cohort A was used primarily to evaluate the efficacy and safety of the combination therapy regimen in the treatment of recurrent or metastatic HNSCC that had failed a first-line platinum-based chemotherapy regimen. Cohort B was used primarily to evaluate the efficacy and safety of the combination therapy regimen in the treatment of PD-L1-positive HNSCC that had not received previous systemic treatment for recurrent or metastatic disease. The cohort for the Phase II study involved in the present disclosure is cohort A.

Test drugs: Anti-PD-1 antibody toripalimab injection (sourced from Suzhou Zhonghe Bio-Pharmaceutical Technology Co., Ltd., strength: 240 mg/6 mL/vial) and cetuximab injection (sourced from Merck, Germany, strength: 100 mg/20 mL/vial).

This study included a screening period, a treatment period, and a follow-up period. The screening period lasted no more than 28 days. After the examinations and evaluations in the screening period were completed, eligible subjects entered the study treatment period. Subjects should receive study treatment as per the protocol until the occurrence of radiologically confirmed progressive disease as judged by the investigator according to the RECIST 1.1 criteria, intolerable toxic response, the subject's voluntary request to discontinue study treatment or withdrawal of informed consent, the investigator's judgment to discontinue treatment in the subject, or toripalimab reaching the maximum treatment duration of 2 years (whichever occurred first).

Safety data were collected continuously throughout the study. Safety and tolerability were evaluated by judging the incidence and severity of adverse events according to the NCI-CTCAE v5.0 criteria. Laboratory examinations, vital signs, ECOG scores, physical examinations, ECGs, and adverse events were continuously evaluated during the trial.

Subject tumor evaluation was conducted at screening (as baseline), every 6 weeks (±7 days) during the first 12 months from the first dose, and then every 9 weeks (±7 days) until radiologically confirmed progressive disease (PD), iCPD as judged by the investigator according to iRECIST (for subjects who showed progressive disease as confirmed by initial radiological findings but were judged by the investigator to be eligible to continue to receive medication), withdrawal of informed consent, loss to follow-up, initiation of new anti-tumor therapy, or discontinuation of the study. Subjects who were excluded for reasons other than progressive disease (including exclusion due to AEs or treatment interval out of visit window) and had not experienced progressive disease at the time of discontinuation were required to continue radiological evaluations until progressive disease, death, loss to follow-up, or initiation of new anti-tumor therapy. The management of subject medication was performed based on the investigator's tumor evaluation results.

### Phase Ib study design

In the Phase Ib study, enrolled subjects received 240 mg of toripalimab administered via intravenous injection once every three weeks. The subjects also needed to receive cetuximab administered via intravenous injection once every week; the dose for the first administration in the first cycle was 400 mg/m², and for the remaining administrations in the first cycle and subsequent cycles, the dose was 250 mg/m² (with an alternative dose of 200 mg/m²). Every three weeks was counted as one treatment cycle. On day 1 of each cycle, toripalimab was administered first for treatment, and after about 60 min of observation, cetuximab was administered for treatment. The specific doses administered are shown in the table below:

| Dose group | Test drug | Each cycle (21 days) | | |
|---|---|---|---|---|
| | | Day 1 | Day 8 | Day 15 |
| Initial dose group | Toripalimab | 240 mg | / | / |
| | Cetuximab | First cycle: 400 mg/m² Subsequent cycles: 250 mg/m² | 250 mg/m² | 250 mg/m² |
| Alternative dose group | Toripalimab | 240 mg | / | / |
| | Cetuximab | First cycle: 400 mg/m² Subsequent cycles: 200 mg/m² | 200 mg/m² | 200 mg/m² |

First, 6 subjects were enrolled to receive treatment at the initial dose, with the first medication cycle (21 days) serving as a tolerability observation period. If fewer than 2 subjects experience DLT, 6 more subjects will be enrolled (for a total of 12 subjects) to collect sufficient safety and PK data for the combination therapy. The Safety Monitoring Committee (SMC) will determine whether to use this dose as the recommended Phase II dose based on data such as the safety and PK results from the 12 subjects.

If 2 or more of the first 6 subjects experience DLT, the dose will be adjusted to the alternative dose, and enrollment and observation will proceed according to the aforementioned enrollment plan. If 2 or more of the first 6 subjects in the alternative dose group experience DLT, the SMC will decide whether to use a different dose level/administration cycle for the study by the SMC, based on data obtained such as the safety and PK results.

### Phase II study design

Subjects enrolled in the Phase II study received treatment with toripalimab in combination with cetuximab at RP2D determined in the Phase Ib. This administration should continue until the occurrence of radiologically confirmed progressive disease as judged by the investigator according to the RECIST 1.1 criteria, intolerable toxic response, the subject's voluntary request to discontinue study treatment or withdrawal of informed consent, the investigator's judgment to discontinue treatment in the subject, or toripalimab reaching the maximum treatment duration of 2 years (whichever occurred first). The planned enrollment for this phase included 12 subjects receiving the same dose as in the Phase Ib.

### 1.2 Enrollment criteria and status

Enrollment criteria:
1. Voluntary participation in this study with fully informed consent and signing of a written informed consent form.
2. Aged between 18 and 75 years at the time of signing the informed consent form.
3. Histologically or cytologically confirmed recurrent or metastatic head and neck squamous cell carcinoma occurring in the oral cavity, oropharynx, hypopharynx, larynx, or paranasal sinuses, deemed unsuitable for local treatment such as surgery or radiotherapy.
4. Phase Ib and Phase II cohort A: Has received a previous first-line platinum-based chemotherapy regimen for recurrent or metastatic disease, with progression during treatment or after completion of treatment; or has received a platinum-based regimen as neoadjuvant or adjuvant chemotherapy (or chemoradiotherapy), with recurrence or metastasis occurring within 6 months after treatment.
5. Phase II cohort B:
1) Has not received previous systemic treatment for recurrent or metastatic disease (if the subject has previously received systemic treatment as part of local treatment, recurrence or metastasis must occur 6 months after completion of treatment).
2) Providing a qualified tumor tissue sample, tested by the central laboratory to be positive for PD-L1 expression ("positive" is defined as a combined positive score (CPS) of ≥ 1).

6. All acute toxic responses caused by previous anti-tumor therapy, surgery or radiotherapy, etc. have alleviated to Grade 0-1 (according to NCI-CTCAE version 5.0) or to levels specified in the enrollment/exclusion criteria (except for hair loss, pigmentation, or other toxicities deemed by the investigator as posing no safety risk to the subject and not affecting treatment compliance).
7. Capable of providing a previous tumor specimen or a fresh tumor tissue biopsy sample.
8. For oropharyngeal carcinoma subjects: capable of providing a previous HPV16 testing report or a qualified tumor tissue sample for testing HPV status.
9. Having a least 1 measurable lesion according to the RECIST 1.1 evaluation criteria.
10. Expected survival ≥ 12 weeks.
11. Eastern Cooperative Oncology Group (ECOG) performance status score of 0 or 1.
12. Good organ function indicators:

| Indicator | Laboratory value |
|---|---|
| Hematology (no blood transfusion or colony-stimulating factor or other medications for correction within 14 days prior to the first study administration) | |
| Neutrophil count | ≥1.5×10⁹/L |
| Platelet count | ≥100×10⁹/L |
| Hemoglobin | ≥90 g/L |

| Renal function | |
|---|---|
| Serum creatinine or creatinine clearance calculated by reference to the Cockcroft-Gault formula or central practice | ≤ 1.5 × upper limit of normal (ULN) |
| | ≤ 3 × ULN (for patients known to suffer from Gilbert's disease) |
| ALT/AST | ≤ 3 × ULN (without liver metastasis) or |
| | ≤ 5 × ULN (if liver metastasis occurs) |
| Albumin (no albumin formulation infused within 14 days prior to the first study administration) | ≥30 g/L |

| Blood coagulation function | |
|---|---|
| International normalized ratio (INR) | ≤ 1.5 × ULN |
| Prothrombin time (PT) | |
| Activated partial thromboplastin time (aPTT) | |

13. Female subjects of childbearing age must confirm a negative serum pregnancy test within 72 h prior to the first dose and agree to take effective contraceptive measures during the use of the study drug and for 60 days following the last dose. In this protocol, a female subject of childbearing age is defined as a sexually mature woman who:
1) has not undergone hysterectomy or bilateral oophorectomy,
2) has not experienced natural menopause for 24 consecutive months (amenorrhea following cancer treatment does not preclude fertility) (i.e., menstruation occurred at any time during the previous 24 consecutive months).

Male patients with female partners of childbearing age must agree to take effective contraceptive measures during the use of the study drug and for 60 days following the last dose.

### Enrollment information:

In the study for Phase II cohort A, a total of 45 patients were enrolled, including 35 male patients (77.8%) and 10 female patients (22.2%). The patients' ages ranged from 32 to 74 years, with a median age of 59 years. Distant metastasis occurred in 18 patients (40%), and 31 patients (68.9%) had a PD-L1 CPS of ≥ 1.

**Table 1: Demographic data of the enrolled subjects**

| Characteristic | Subjects (N = 45), n (%) |
|---|---|
| Age, median (range) | 59 (32-74) |
| Gender: male/female | 35 (77.8%)/10 (22.2%) |
| Distant metastasis | 18 (40%) |
| PD-L1 CPS≥1 | 31 (68.9%) |
| ECOG: 0/1 | 8(17.8%)/37 (82.2%) |

### 1.3 Study content and results

### 1.3.1 Safety study

### Study methodology:

Safety analysis was conducted on the safety analysis set.

The extent of exposure of each study drug was summarized based on the number of treatment cycles (number and proportion of patients), duration of medication (days), cumulative total dose (mg) received by each patient, dose intensity, and relative dose intensity.

Adverse events were coded and classified using MedDRA25.1. The number and incidence of treatment-related adverse events (TRAEs) occurring during the treatment period were tabulated by preferred terminology and system organ classification. In addition, severe adverse events, Grade 3 or higher adverse events, adverse events related to the study drug, and adverse events leading to discontinuation or interruption of administration of the study drug were also summarized accordingly. Multiple occurrences of the same event were counted once at the highest severity. The afore-mentioned types of adverse events are shown in the table.

The deaths reported during the study treatment period and during the follow-up period after termination of the treatment were summarized.

Descriptive statistics were used to summarize the measurements of laboratory examinations, physical examinations, vital signs, body weight, ECOG performance status, ECG, etc. at baseline and the last visit during the treatment period, as well as the relative changes from baseline, and the number and proportion of patients exhibiting abnormal changes post-baseline. The measurements for each visit are also shown in the table.

### Study results:

No DLT was observed in the Phase Ib study, so the initial dose in the Phase Ib study was used as the recommended dose for the Phase II trial. As of March 15, 2023, the median follow-up time for patients in Cohort A was 9.6 months. All 45 patients (100%) experienced TEAEs. TRAEs occurred in 41 patients (91.1%), with the most common being rash (35.6%), hypomagnesemia (17.8%), hypothyroidism (17.8%), acneiform dermatitis (15.6%), and paronychia (15.6%). Grade 3 or higher TRAEs occurred in 10 patients (22.2%), including oral mucositis, hypomagnesemia, decreased white blood cell count, hypokalemia, lacunar cerebral infarction, soft tissue infection, infusion-related reactions, and gastric perforation. irAEs occurred in 11 patients (24.4%), including immune-related adverse skin reactions, hypothyroidism, thyroid dysfunction, duodenitis, peripheral edema, and immune-mediated arthritis. No Grade 3 or higher irAE occurred. Administration of cetuximab was permanently discontinued in 1 patient due to a Grade 3 infusion reaction, and administration of both toripalimab and cetuximab was permanently discontinued in another patient due to a Grade 5 gastric perforation. AEs leading to death occurred in 7 patients (15.6%), in which 1 case of gastric perforation was evaluated by the investigator to be related to the study treatment, while the others were evaluated by the investigator to be unrelated to the study treatment, including hemorrhagic shock, pulmonary embolism, tumor hemorrhage (2 cases), and myocardial infarction. Infusion reactions occurred in 7 patients (15.6%), in which 2 patients (4.4%) experienced infusion reactions related to toripalimab, while 5 patients (11.1%) experienced infusion reactions related to cetuximab. One patient (2.2%) experienced a Grade 3 infusion reaction related to cetuximab. No Grade 4 or 5 infusion reaction was observed.

**Table 2: Summary of occurred adverse events (N = 45)**

| | N | Incidence (%) |
|---|---|---|
| TEAE | 45 | 100 |
| TRAE | 41 | 91.1 |
| Grade 3 or higher TRAE | 10 | 22.2 |
| TRAE leading to permanent discontinuation of administration of any drug | 2 | 4.4 |
| irAE | 11 | 24.4 |
| Grade 3 or higher irAE | 0 | 0 |
| TEAE leading to death | 7 | 15.6 |
| TRAE leading to death | 1 | 2.2 |
| Infusion reaction | 7 | 15.6 |
| Grade 3 or higher infusion reaction | 1 | 2.2 |

### 1.3.2 Anti-tumor activity study

### Study methodology:

The primary endpoint of this study was the ORR. The efficacy data were analyzed based on the full analysis set and the evaluable efficacy analysis set. The objective response rate was analyzed, and the 95% confidence interval for the ORR was calculated using the Clopper-Pearson method.

The median DoR, PFS, and OS were calculated using the Kaplan-Meier method, and their 95% CIs were estimated using the Brookmeyer-Crowley method where the log-log function transformation was used to achieve normal approximation. The 1-year OS rate was estimated using the Kaplan-Meier method, and its 95% confidence interval was estimated using the Greenwood formula.

### Study results:

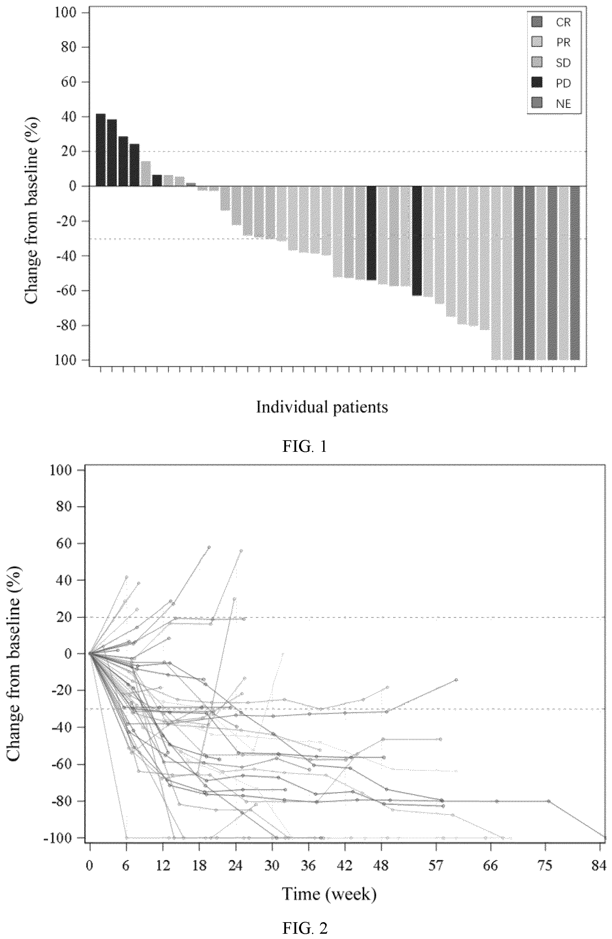

As of March 15, 2023, based on the investigator's evaluations, 22 patients were confirmed to have achieved PR or CR (ORR 48.9%), and 13 patients achieved SD (DCR 77.8%) (as shown in FIGs. 1 and 2). Among them, patients with PD-L1 CPS ≥ 1 exhibited superior ORR values (51.6% vs 40%) and median DOR values (546 days vs 127 days) compared to those with PD-L1 CPS < 1.

**Table 3: Summary of efficacy**

| | |
|---|---|
| Tumor response evaluation | Subjects (N = 45), n (%) |
| Complete response (CR) | 4(8.9%) |
| Partial response (PR) | 18(40%) |
| Stable disease (SD) | 13(28.9%) |
| Progressive disease (PD) | 7(15.6%) |
| Not evaluable (NE) | 3(6.7%) |
| ORR (95%CI) | 48.9% (33.7%-64.2%) |
| DCR (95%CI) | 77.8% (62.9%-88.8%) |

**Table 4: Summary of efficacy in patients with different PD-L1 status**

| PD-L1 status | DCR (95%CI) | ORR (95%CI) | Median DOR (day) |
|---|---|---|---|
| CPS<1 | 80 (44.4, 97.5) | 40 (12.2, 73.8) | 127 (N=4) |
| CPS≥1 | 77.2 (58.9, 90.4) | 51.6 (33.1, 69.9) | 546 (N=16) |
| Overall population | 77.8 (62.9, 88.8) | 48.9 (33.7, 64.2) | 546 (N=22) |

| | | | |
|---|---|---|---|
| Note: The PD-L1 CPS results for 4 patients were NA. | | | |

### 1.3.3 Study conclusion

According to the study results, toripalimab in combination with cetuximab demonstrated good tolerability and initial excellent clinical benefits in the treatment of recurrent or metastatic head and neck squamous cell carcinoma (HNSCC) that had failed the first-line platinum-based treatment regimen.

## Claims

1. A pharmaceutical combination, comprising an anti-PD-1 antibody or an antigen-binding fragment thereof and an anti-EGFR antibody or an antigen-binding fragment thereof.

2. The pharmaceutical combination according to claim 1, wherein the anti-PD-1 antibody or the antigen-binding fragment thereof comprises LCDR1 with the amino acid sequence set forth in SEQ ID NO: 1 or an amino acid sequence having 1, 2, or 3 amino acid differences compared to SEQ ID NO: 1, LCDR2 with the amino acid sequence set forth in SEQ ID NO: 2 or an amino acid sequence having 1, 2, or 3 amino acid differences compared to SEQ ID NO: 2, LCDR3 with the amino acid sequence set forth in SEQ ID NO: 3 or an amino acid sequence having 1, 2, or 3 amino acid differences compared to SEQ ID NO: 3, HCDR1 with the amino acid sequence set forth in SEQ ID NO: 4 or an amino acid sequence having 1, 2, or 3 amino acid differences compared to SEQ ID NO: 4, HCDR2 with the amino acid sequence set forth in SEQ ID NO: 5 or an amino acid sequence having 1, 2, or 3 amino acid differences compared to SEQ ID NO: 5, and HCDR3 with the amino acid sequence set forth in SEQ ID NO: 6 or an amino acid sequence having 1, 2, or 3 amino acid differences compared to SEQ ID NO: 6;
preferably, the anti-PD-1 antibody or the antigen-binding fragment thereof comprises a light chain variable region having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 7, and a heavy chain variable region having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 8;
more preferably, the anti-PD-1 antibody or the antigen-binding fragment thereof comprises a light chain variable region with the amino acid sequence set forth in SEQ ID NO: 7 and a heavy chain variable region with the amino acid sequence set forth in SEQ ID NO: 8.

3. The pharmaceutical combination according to claim 2, wherein the anti-PD-1 antibody or the antigen-binding fragment thereof comprises a light chain having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 9, and a heavy chain having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 10;
preferably, the anti-PD-1 antibody comprises a light chain with the amino acid sequence set forth in SEQ ID NO: 9 and a heavy chain with the amino acid sequence set forth in SEQ ID NO: 10.

4. The pharmaceutical combination according to claim 1, wherein the anti-PD-1 antibody is selected from one or more of nivolumab or a biosimilar thereof, pembrolizumab or a biosimilar thereof, toripalimab or a biosimilar thereof, sintilimab or a biosimilar thereof, camrelizumab or a biosimilar thereof, tislelizumab or a biosimilar thereof, cemiplimab or a biosimilar thereof, zimberelimab or a biosimilar thereof, penpulimab or a biosimilar thereof, and serplulimab or a biosimilar thereof, preferably toripalimab or a biosimilar thereof.

5. The pharmaceutical combination according to any one of claims 1-4, wherein the anti-EGFR antibody or the antigen-binding fragment thereof comprises LCDR1 with the amino acid sequence set forth in SEQ ID NO: 11 or an amino acid sequence having 1, 2, or 3 amino acid differences compared to SEQ ID NO: 11, LCDR2 with the amino acid sequence set forth in SEQ ID NO: 12 or an amino acid sequence having 1, 2, or 3 amino acid differences compared to SEQ ID NO: 12, LCDR3 with the amino acid sequence set forth in SEQ ID NO: 13 or an amino acid sequence having 1, 2, or 3 amino acid differences compared to SEQ ID NO: 13, HCDR1 with the amino acid sequence set forth in SEQ ID NO: 14 or an amino acid sequence having 1, 2, or 3 amino acid differences compared to SEQ ID NO: 14, HCDR2 with the amino acid sequence set forth in SEQ ID NO: 15 or an amino acid sequence having 1, 2, or 3 amino acid differences compared to SEQ ID NO: 15, and HCDR3 with the amino acid sequence set forth in SEQ ID NO: 16 or an amino acid sequence having 1, 2, or 3 amino acid differences compared to SEQ ID NO: 16.

6. The pharmaceutical combination according to claim 5, wherein the anti-EGFR antibody or the antigen-binding fragment thereof comprises a light chain variable region having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 17, and a heavy chain variable region having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 18;
preferably, the anti-EGFR antibody or the antigen-binding fragment thereof comprises a light chain variable region with the amino acid sequence set forth in SEQ ID NO: 17 and a heavy chain variable region with the amino acid sequence set forth in SEQ ID NO: 18.

7. The pharmaceutical combination according to claim 6, wherein the anti-EGFR antibody or the antigen-binding fragment thereof comprises a light chain having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 19, and a heavy chain having at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO: 20;
preferably, the anti-EGFR antibody comprises a light chain with the amino acid sequence set forth in SEQ ID NO: 19 and a heavy chain with the amino acid sequence set forth in SEQ ID NO: 20.

8. The pharmaceutical combination according to any one of claims 1-4, wherein the anti-EGFR antibody is selected from one or more of necitumumab or a biosimilar thereof, nimotuzumab or a biosimilar thereof, panitumumab or a biosimilar thereof, and cetuximab or a biosimilar thereof, preferably cetuximab or a biosimilar thereof.

9. The pharmaceutical combination according to any one of claims 1-8, wherein the pharmaceutical combination comprises the anti-PD-1 antibody or the antigen-binding fragment thereof in an amount sufficient for administration at a frequency of about once every week, once every two weeks, once every three weeks, once every four weeks, or once a month, preferably once every three weeks, and the anti-EGFR antibody or the antigen-binding fragment thereof in an amount sufficient for administration at a frequency of about once every week, once every two weeks, once every three weeks, once every four weeks, or once a month, preferably once every week;
preferably, in the pharmaceutical combination, the amount of the anti-PD-1 antibody or the antigen-binding fragment thereof is sufficient for administration at the following single doses: about 0.1 mg/kg individual body weight to about 10.0 mg/kg individual body weight, preferably about 1.0 mg/kg individual body weight to about 10.0 mg/kg individual body weight, such as about 0.1 mg/kg individual body weight, about 0.3 mg/kg individual body weight, about 1 mg/kg individual body weight, about 2 mg/kg individual body weight, about 3 mg/kg individual body weight, about 5 mg/kg individual body weight, or about 10 mg/kg individual body weight, or a fixed dose of about 120 mg to about 480 mg, preferably a fixed dose of about 120 mg to about 360 mg, such as a fixed dose of about 120 mg, about 240 mg, about 360 mg, or about 480 mg, preferably a fixed dose of about 240 mg; and the amount of the anti-EGFR antibody or the antigen-binding fragment thereof is sufficient for administration at the following single doses: about 100 mg/m² individual body surface area to about 500 mg/m² individual body surface area, preferably 200 mg/m² individual body surface area to about 500 mg/m² individual body surface area, such as about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area, preferably about 200 mg/m² individual body surface area, about 250 mg/m² individual body surface area, about 300 mg/m² individual body surface area, about 350 mg/m² individual body surface area, or about 400 mg/m² individual body surface area;
preferably, the pharmaceutical combination comprises 1 dose of the anti-PD-1 antibody or the antigen-binding fragment thereof and 3 doses of the anti-EGFR antibody or the antigen-binding fragment thereof, wherein the anti-PD-1 antibody or the antigen-binding fragment thereof and the anti-EGFR antibody or the antigen-binding fragment thereof are provided in separate formulations;
preferably, the 1 dose of the formulation of the anti-PD-1 antibody or the antigen-binding fragment thereof comprises 240 mg of the anti-PD-1 antibody or the antigen-binding fragment thereof, and among the 3 doses of the formulation of the anti-EGFR antibody or the antigen-binding fragment thereof, 1 dose of the formulation comprises the anti-EGFR antibody or the antigen-binding fragment thereof sufficient for administration at a daily dose of 400 mg/m², while the other 2 doses are sufficient for administration at a daily dose of 250 mg/m², or each of the 3 doses of the formulation of the anti-EGFR antibody or the antigen-binding fragment thereof comprises the anti-EGFR antibody or the antigen-binding fragment thereof sufficient for administration at a daily dose of 250 mg/m²;
preferably, the anti-PD-1 antibody is toripalimab or an antigen-binding fragment thereof, and the anti-EGFR antibody is cetuximab or an antigen-binding fragment thereof.

10. Use of the pharmaceutical combination according to any one of claims 1-9 in the preparation of a medicament for preventing or treating head and neck squamous cell carcinoma (HNSCC) in a patient.

11. The use according to claim 10, wherein the head and neck squamous cell carcinoma is recurrent or metastatic head and neck squamous cell carcinoma, preferably recurrent or metastatic head and neck squamous cell carcinoma that has failed a previous first-line platinum-based chemotherapy regimen; preferably, the patient has a PD-L1 CPS of ≥ 1.

12. The use according to claim 10 or 11, wherein the head and neck squamous cell carcinoma has ≥ 1 measurable lesion according to RECIST v1.1 criteria.

13. Use of an anti-PD-1 antibody or an antigen-binding fragment thereof in the preparation of a medicament or kit for preventing or treating head and neck squamous cell carcinoma (HNSCC) in a patient, or use of a combination of an anti-PD-1 antibody or an antigen-binding fragment thereof and an anti-EGFR antibody or an antigen-binding fragment thereof in the preparation of a medicament or kit for preventing or treating head and neck squamous cell carcinoma in a patient, wherein preferably, the anti-PD-1 antibody or the antigen-binding fragment thereof is according to any one of claims 2-4; preferably, the anti-EGFR antibody or the antigen-binding fragment thereof is according to any one of claims 5-8.

14. The use according to claim 13, wherein the head and neck squamous cell carcinoma is recurrent or metastatic head and neck squamous cell carcinoma, preferably recurrent or metastatic head and neck squamous cell carcinoma that has failed a previous first-line platinum-based chemotherapy regimen; preferably, the patient has a PD-L1 CPS of ≥ 1.

15. The use according to claim 13 or 14, wherein the head and neck squamous cell carcinoma has ≥ 1 measurable lesion according to RECIST v1.1 criteria.

16. The use according to any one of claims 13-15, wherein
in the use of the anti-PD-1 antibody or the antigen-binding fragment thereof in the preparation of a medicament or kit for preventing or treating head and neck squamous cell carcinoma (HNSCC), the anti-PD-1 antibody or the antigen-binding fragment thereof is administered at a single dose of about 0.1 mg/kg individual body weight to about 10.0 mg/kg individual body weight, preferably about 1.0 mg/kg individual body weight to about 10.0 mg/kg individual body weight, such as about 0.1 mg/kg individual body weight, about 0.3 mg/kg individual body weight, about 1 mg/kg individual body weight, about 2 mg/kg individual body weight, about 3 mg/kg individual body weight, about 5 mg/kg individual body weight, or about 10 mg/kg individual body weight, or of a fixed dose of about 120 mg to about 480 mg, preferably a fixed dose of about 120 mg to about 360 mg, such as a fixed dose of about 120 mg, about 240 mg, about 360 mg, or about 480 mg;
in the use of the combination of the anti-PD-1 antibody or the antigen-binding fragment thereof and the anti-EGFR antibody or the antigen-binding fragment thereof in the preparation of a medicament or kit for preventing or treating head and neck squamous cell carcinoma, the anti-PD-1 antibody or the antigen-binding fragment thereof is administered at a single dose of about 0.1 mg/kg individual body weight to about 10.0 mg/kg individual body weight, preferably about 1.0 mg/kg individual body weight to about 10.0 mg/kg individual body weight, such as about 0.1 mg/kg individual body weight, about 0.3 mg/kg individual body weight, about 1 mg/kg individual body weight, about 2 mg/kg individual body weight, about 3 mg/kg individual body weight, about 5 mg/kg individual body weight, or about 10 mg/kg individual body weight, or of a fixed dose of about 120 mg to about 480 mg, preferably a fixed dose of about 120 mg to about 360 mg, such as a fixed dose of about 120 mg, about 240 mg, about 360 mg, or about 480 mg, preferably a fixed dose of about 240 mg; and the anti-EGFR antibody or the antigen-binding fragment thereof is administered at a single dose of about 100 mg/m² individual body surface area to about 500 mg/m² individual body surface area, preferably 200 mg/m² individual body surface area to about 500 mg/m² individual body surface area, such as about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area, preferably about 200 mg/m² individual body surface area, about 250 mg/m² individual body surface area, about 300 mg/m² individual body surface area, about 350 mg/m² individual body surface area, or about 400 mg/m² individual body surface area.

17. The use according to any one of claims 13-16, wherein
in the use of the anti-PD-1 antibody or the antigen-binding fragment thereof in the preparation of a medicament or kit for preventing or treating head and neck squamous cell carcinoma (HNSCC), the anti-PD-1 antibody or the antigen-binding fragment thereof is administered at a frequency of about once every week, once every two weeks, once every three weeks, once every four weeks, or once a month, preferably once every two weeks or once every three weeks;
in the use of the combination of the anti-PD-1 antibody or the antigen-binding fragment thereof and the anti-EGFR antibody or the antigen-binding fragment thereof in the preparation of a medicament or kit for preventing or treating head and neck squamous cell carcinoma, the anti-PD-1 antibody or the antigen-binding fragment thereof is administered at a frequency of about once every week, once every two weeks, once every three weeks, once every four weeks, or once a month, preferably once every three weeks, and the anti-EGFR antibody or the antigen-binding fragment thereof is administered at a frequency of about once every week, once every two weeks, once every three weeks, once every four weeks, or once a month, preferably once every week.

18. The use according to any one of claims 13-16, wherein
in the use of the anti-PD-1 antibody or the antigen-binding fragment thereof in the preparation of a medicament or kit for preventing or treating head and neck squamous cell carcinoma (HNSCC), the anti-PD-1 antibody or the antigen-binding fragment thereof is administered once every two or three weeks, at a dose of about 1 mg/kg individual body weight, about 3 mg/kg individual body weight, about 5 mg/kg individual body weight, or about 10 mg/kg individual body weight, or of a fixed dose of about 240 mg, a fixed dose of about 360 mg, or a fixed dose of about 480 mg;
in the use of the combination of the anti-PD-1 antibody or the antigen-binding fragment thereof and the anti-EGFR antibody or the antigen-binding fragment thereof in the preparation of a medicament or kit for preventing or treating head and neck squamous cell carcinoma, the anti-PD-1 antibody or the antigen-binding fragment thereof is administered once every three weeks at a fixed dose of about 240 mg, and the anti-EGFR antibody or the antigen-binding fragment thereof is administered once every week, at a dose of about 400 mg/m² individual body surface area in the first cycle, and about 250 mg/m² individual body surface area in each subsequent administration cycle.

19. The use according to any one of claims 10-18, wherein
in the use of the anti-PD-1 antibody or the antigen-binding fragment thereof in the preparation of a medicament or kit for preventing or treating head and neck squamous cell carcinoma (HNSCC), the anti-PD-1 antibody or the antigen-binding fragment thereof can be administered in cycles of one week, two weeks, three weeks, one month, two months, three months, four months, five months, half a year, one year, two years, or longer; optionally, the administration cycles have identical or different durations, and are at identical or different intervals; or
in the use of the combination of the anti-PD-1 antibody or the antigen-binding fragment thereof and the anti-EGFR antibody or the antigen-binding fragment thereof in the preparation of a medicament or kit for preventing or treating head and neck squamous cell carcinoma, the anti-PD-1 antibody or the antigen-binding fragment thereof and the anti-EGFR antibody or the antigen-binding fragment thereof can be administered in cycles of one week, two weeks, three weeks, one month, two months, three months, four months, five months, half a year, one year, two years, or longer; optionally, the administration cycles have identical or different durations, and are at identical or different intervals.

20. The use according to any one of claims 10-19, wherein
in the use of the anti-PD-1 antibody or the antigen-binding fragment thereof in the preparation of a medicament or kit for preventing or treating head and neck squamous cell carcinoma (HNSCC), the anti-PD-1 antibody or the antigen-binding fragment thereof is administered in a liquid dosage form such as an injection, via a parenteral route;
in the use of the combination of the anti-PD-1 antibody or the antigen-binding fragment thereof and the anti-EGFR antibody or the antigen-binding fragment thereof in the preparation of a medicament or kit for preventing or treating head and neck squamous cell carcinoma, the anti-PD-1 antibody or the antigen-binding fragment thereof and the anti-EGFR antibody or the antigen-binding fragment thereof are administered in a liquid dosage form such as an injection, via a parenteral route.

21. A kit, comprising:
one or more single dosage units of an anti-PD-1 antibody or an antigen-binding fragment thereof, wherein preferably, the anti-PD-1 antibody or the antigen-binding fragment thereof is according to any one of claims 2-4; or
one or more single dosage units of an anti-PD-1 antibody or an antigen-binding fragment thereof and one or more single dosage units of an anti-EGFR antibody or an antigen-binding fragment thereof, wherein preferably, the anti-PD-1 antibody or the antigen-binding fragment thereof is according to any one of claims 2-4, and preferably, the anti-EGFR antibody or the antigen-binding fragment thereof is according to any one of claims 5-8.

22. The kit according to claim 18, comprising:
(I) one or more single dosage units of the anti-PD-1 antibody or the antigen-binding fragment thereof, wherein the single dosage unit comprises the anti-PD-1 antibody or the antigen-binding fragment thereof in an amount of a fixed dose of about 120 mg to about 480 mg, such as a fixed dose of about 120 mg, a fixed dose of about 240 mg, a fixed dose of about 360 mg, or a fixed dose of about 480 mg, or comprises the anti-PD-1 antibody or the antigen-binding fragment thereof in an amount sufficient for administration to a patient at a single dose of about 1.0 mg/kg individual body weight to about 10.0 mg/kg individual body weight, such as about 1 mg/kg individual body weight, about 3 mg/kg individual body weight, about 5 mg/kg individual body weight, or about 10 mg/kg individual body weight; or
(II) one or more single dosage units of the anti-PD-1 antibody or the antigen-binding fragment thereof, wherein the single dosage unit comprises the anti-PD-1 antibody or the antigen-binding fragment thereof in an amount of a fixed dose of about 120 mg to about 480 mg, preferably a fixed dose of about 120 mg to about 360 mg, such as about 120 mg, about 240 mg, about 360 mg, or about 480 mg, preferably a fixed dose of about 240 mg; and one or more single dosage units of the anti-EGFR antibody or the antigen-binding fragment thereof, wherein the single dosage unit comprises the anti-EGFR antibody or the antigen-binding fragment thereof in an amount sufficient for administration to a patient at a single dose of about 100 mg/m² individual body surface area to about 500 mg/m² individual body surface area, preferably 200 mg/m² individual body surface area to about 500 mg/m² individual body surface area, such as about 100 mg/m² individual body surface area, about 150 mg/m² individual body surface area, about 200 mg/m² individual body surface area, about 250 mg/m² individual body surface area, about 300 mg/m² individual body surface area, about 350 mg/m² individual body surface area, about 400 mg/m² individual body surface area, about 450 mg/m² individual body surface area, or about 500 mg/m² individual body surface area, preferably about 200 mg/m² individual body surface area, about 250 mg/m² individual body surface area, about 300 mg/m² individual body surface area, about 350 mg/m² individual body surface area, or about 400 mg/m² individual body surface area.

23. The kit according to claim 18, wherein the kit comprises 1 or more pharmaceutical combinations according to any one of claims 1-9.
